# EUROPEAN PATENT SPECIFICATION

(11) **EP 1 455 821 B1**
(45) Date of publication and mention of the grant of the patent: **08.12.2010**
(21) Application number: 02752184.8
(22) Date of filing: 03.07.2002
(51) Int. Cl.: A61K 39/395, C07K 16/26, C12P 21/08, G01N 33/53, G01N 33/543, G01N 33/74

(54) **PARATHYROID HORMONE ANTIBODIES AND RELATED METHODS**
PARATHORMON-ANTIKÖRPER UND DAMIT ZUSAMMENHÄNGENDE VERFAHREN
ANTICORPS D'HORMONE PARATHYROIDE ET PROCEDES ASSOCIES

(30) Priority: 03.07.2001 US 898398
(43) Date of publication of application: 15.09.2004
(73) Proprietor: Quest Diagnostics Investments Incorporated, Wilmington, DE 19899 (US)
(72) Inventor: HUTCHISON, James, Scott, Newport Beach, CA 92657 (US)
(74) Representative: Kühn, Armin
(86) International application number: PCT/US2002/021356
(87) International publication number: WO 2003/003986

(56) References cited:
- WO-A-01/44818
- US-A- 6 030 790
- TAMPE J ET AL: "CHARACTERIZATION OF ANTIBODIES AGAINST HUMAN N-TERMINAL PARATHYROID HORMONE BY EPITOPE MAPPING" JOURNAL OF IMMUNOASSAY, MARCEL DEKKER, BASEL, CH, vol. 13, no. 1, 1992, pages 1-13, XP001014389 ISSN: 0197-1522
- MAEGERLEIN M ET AL: "PRODUCTION OF SEQUENCE SPECIFIC POLYCLONAL ANTIBODIES TO HUMAN PARATHYROID HORMONE 1-37 BY IMMUNIZATION WITH MULTIPLE ANTIGENIC PEPTIDES" ARZNEIMITTEL FORSCHUNG. DRUG RESEARCH, ECV EDITIO CANTOR VERLAG, AULENDORF, DE, vol. 48, no. 7, July 1998 (1998-07), pages 783-787, XP001010447 ISSN: 0004-4172
- KURONEN I. ET AL.: 'Hen egg yolk antibodies purified by antigen affinity under highly alkaline conditions provide new tools for diagnostics. Human intact parathyrin as a model antigen' EUR. J. OF CLINICAL CHEMISTRY AND CLINICAL BIOCHEMISTRY vol. 35, no. 6, 1997, pages 435 - 440, XP009062059
- BROWN R.C. ET AL.: 'Comparison of poly- and monoclonal antibodies as labels in a two-site immunochemiluminometric assay for intact parathyoid hormone' JOURNAL OF IMMUNOLOGICAL METHODS vol. 109, 1988, pages 139 - 144, XP002976114
- GAO P. ET AL.: 'Measuring the biologically active or authentic whole parathyroid hormone (PTH) with a novel immunoradiometric assay without cross-reaction to the PTH(7-84) fragment' JOURNAL OF BONE AND MINERAL RESEARCH vol. 14, no. SUPPL.1, September 1999, page S446, XP002976115
- GAO P. ET AL.: 'Immunochemiluminometric assay with two mooclonal antibodies against the N-terminal sequence of human parathyroid hormone' CLINICA CHIMICA ACTA vol. 245, 1996, pages 39 - 59, XP002928303
- JOHN M.R. ET AL.: 'A novel immunoradiometric assay detects full-length human PTH but not amino-terminally truncated fragments: implications for PTH measurements in renal failure' JOURNAL OF CLINICAL ENDOCRINOLOGY AND METABOLISM vol. 84, no. 11, 1999, pages 4287 - 4290, XP001014437
- 'Intact-PTH [parathyroid hormone] ELISA [enzyme-linked immunosorbent assay] Specific quantitative assay for the determination of intact parathyroid hormone in serum' IMMUTOPICS INTERNATIONAL 23 December 1997, pages 1 - 13, XP002976116
- JIN L. ET AL.: 'Crystal structure of human parathyroid hormone 1-34 at 0.9-A resolution' JOURNAL OF BIOLOGICAL CHEMISTRY vol. 275, no. 35, 01 September 2000, pages 27238 - 27244, XP002976117

## Description

### BACKGROUND

Tampe et al. (J. Immunoassay (1992) 13(1):1-13) disclose antisera and monoclonal antibodies raised against human parathyroid hormone (hPTH) 1-84 and hPTH 1-38, respectively. These antisera and monoclonal antibodies were subsequently characterized by epitope mapping using all possible hexapeptide fragments of hPTH 1-34 to identify preferred binding sites for the antibodies in the hPTH molecule. The epitope mapping approach demonstrated that the antibodies contained in the antiserum bind predominantly to the hexapeptides 7-12, 8-13, 9-14 and 10-15 of hPTH.

Maegerlein et al. (Drug Research (1998) 48(7):783-787) disclose antisera raised against fragments of parathyroid hormone, including the peptides hPTH 1-10, hPTH 9-18 and hPTH 24-37. The authors report that one of the antisera raised against hPTH, namely that raised against hPTH 1-10, was able to differentiate between N-terminally intact (1-37) and truncated (3-37 and 4-37) hPTH fragments.

US 6,030,790 discloses hPTH fragments that may be used to generate antibodies that allow the differentiation between biologically active and inactive hPTH and claims antibodies that bind selectively to hPTH 1-10, 1-9, 1-8, 1-7, 1-6 and 1-5.

WO 01/44818 describes an assay that allows the determination of the biological activity of hPTH comprising the use of an antibody that specifically recognizes if the 3 N-terminal amino acids of hPTH are present. The antibody was generated by immunization with hPTH 1-6 and the subsequent separation of antibodies directed against hPTH 3-6

### SUMMARY OF THE INVENTION

The present invention provides antibodies that specifically recognize a three-dimensional epitope of parathyroid hormone comprising amino acids located between amino acids 1-13 of SEQ ID NO:1 and including the first N-terminal amino acid of native PTH along with the intact helix of the amino terminus and methods for using same.

The antibodies recognize and bind the first thirteen amino acids of human parathyroid hormone. In one embodiment, the antibodies of the invention are substantially not crossreactive with non-bioactive parathyroid hormone.

The invention also provides an antibody (monoclonal or polyclonal), and a purified preparation of an antibody, which is capable of forming an immune complex with a three-dimensional epitope of parathyroid hormone comprising amino acids located between amino acids 1-13 of SEQ ID NO:1 and including the first N-terminal amino acid of native PTH along with the intact helix of the amino terminus, such as a human parathyroid hormone, such antibody being generated by using as an antigen, parathyroid hormone, or a variant thereof. This antibody is preferably capable of neutralizing (i.e., partially or completely inhibiting) a biological activity of the parathyroid hormone (i.e., a component of one of the cascades naturally triggered by the parathyroid hormone binding to its receptor). In preferred embodiments, the antibody of the invention is capable of forming an immune complex with parathyroid hormone and is capable of neutralizing a biological activity of the PTH receptor (i.e. adenylate cyclase activation or phospholipase C stimulation).

Also within the invention is a therapeutic composition including, in a pharmaceutically-acceptable carrier, an antibody to parathyroid hormone, or a derivative thereof. These therapeutic compositions provide a means for treating various disorders characterized by overstimulation of parathyroid hormone receptors by a parathyroid hormone. These antibodies are useful as diagnostics, such as for distinguishing those cases of hypercalcemia related to PTH from those which are not.

The antibody of the present invention recognizes any of the above-mentioned peptides, for example human parathyroid hormone.

The antibody recognizes an amino acid sequence from Ser at position 1 to Leu at position 13 of SEQ ID No. 1 or a portion, or portions, included in the amino acid sequence.

In one embodiment, the antibody is a polyclonal antibody or a monoclonal antibody.

The method for measuring parathyroid hormone or a variant thereof disclosed herein includes the steps of: incubating a mixture of a sample and a first antibody recognizing the parathyroid hormone, or variant thereof; adding a labeled second antibody recognizing the parathyroid hormone, or variant thereof, to the mixture, followed by further incubation; and detecting the resulting antigen-antibody complex in the mixture.

Alternatively, the method for measuring parathyroid hormone or a variant thereof disclosed herein includes the steps of: incubating a mixture of a sample, a first antibody recognizing the parathyroid hormone, or variant thereof, and a labeled second antibody recognizing the parathyroid hormone, or variant thereof; and detecting the resulting antigen-antibody complex in the mixture.

The immunological assay for a peptide of the present invention comprises the steps of: incubating a sample including any of the above-mentioned peptides with any of the above-mentioned antibodies under conditions for forming an antigen-antibody complex; and quantifying the antigen-antibody complex.

The kit for an immunological assay of the bioactive forms of parathyroid hormone includes any of the above-mentioned antibodies.

Additional advantages and aspects of the present invention are apparent in the following detailed description and claims.

### BRIEF DESCRIPTION OF THE FIGURES

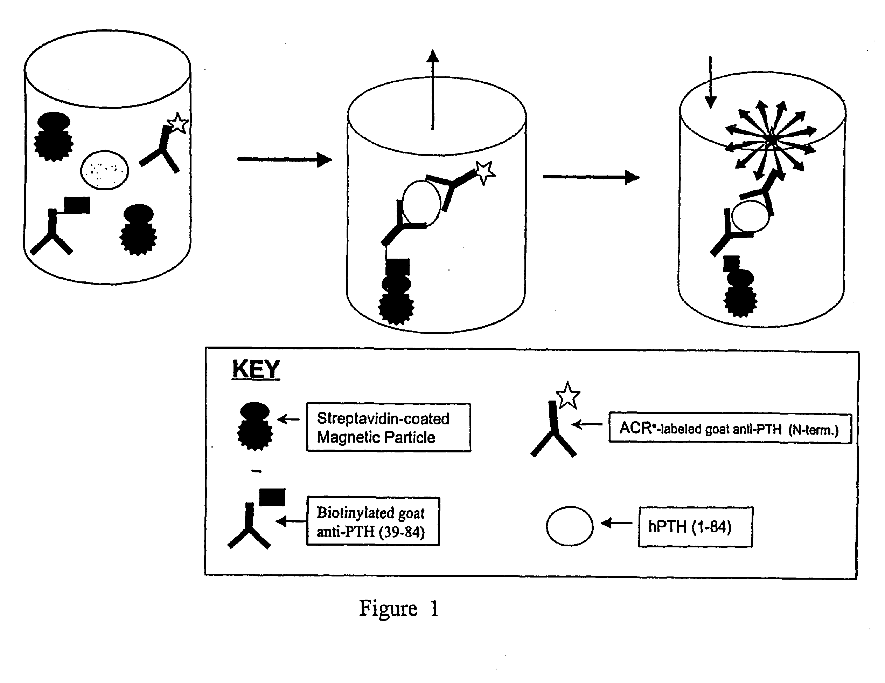

FIG. 1 is a schematic illustration of the procedures used according to the invention.

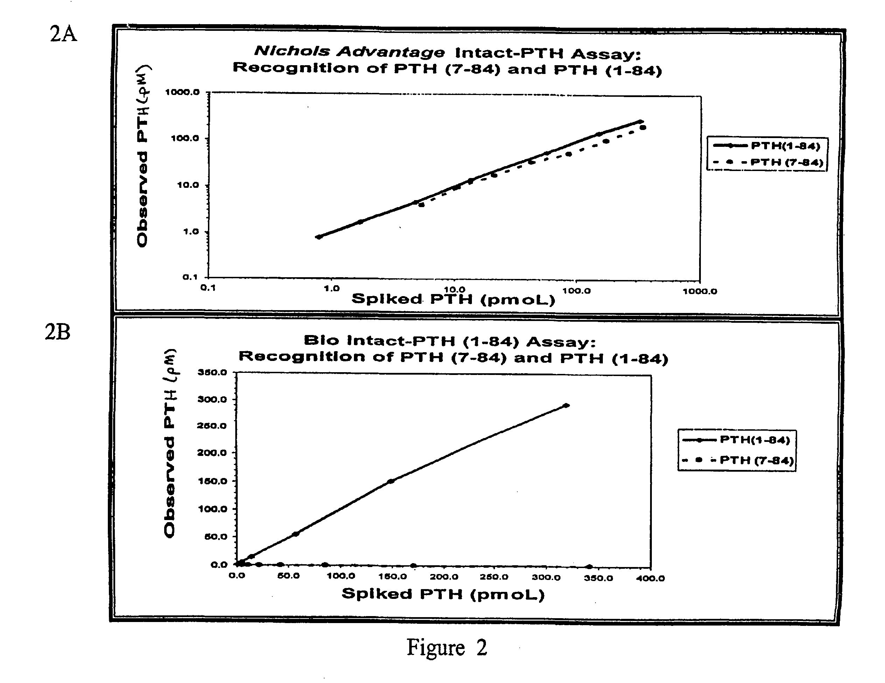

FIG. 2 depicts graphs of observed PTH concentration (picomoles) versus spiked PTH (picomoles). Spiked PTH is defined as the addition of the specified amount of PTH in picomoles into the assay reaction. FIG. 2A illustrates the Nichols Advantage Intact-PTH assay. FIG. 2B illustrates the bio-intact PTH assay using the antibodies of the invention.

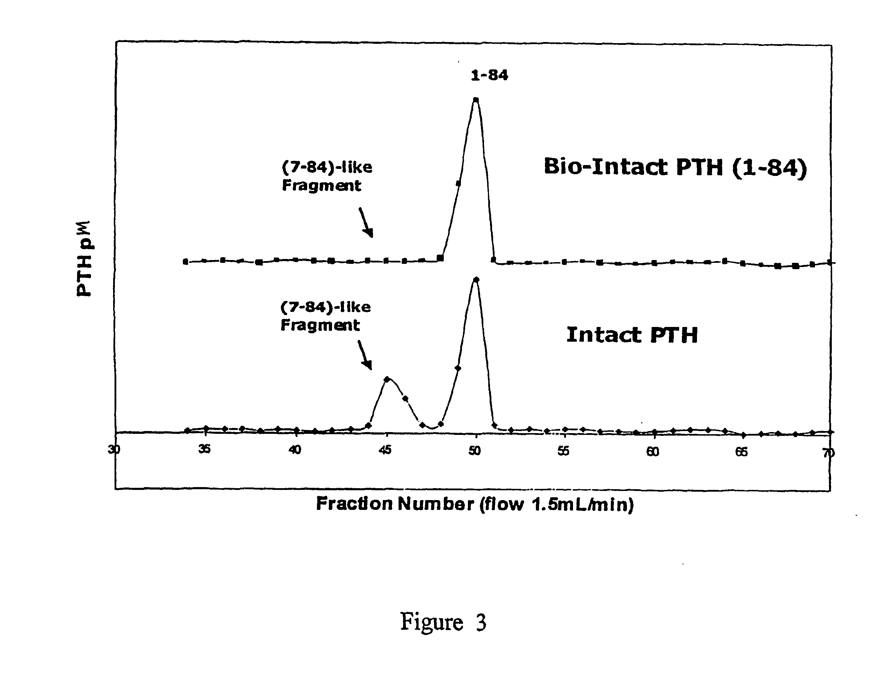

FIG. 3 is a graph of PTH concentration (picomoles) versus fractionation number for bio-intact PTH (using the antibodies of the invention) and intact PTH (using the Nichols Advantage Intact PTH assay). Note the absence of the peak corresponding to the fragment, PTH₇₋₈₄, using the antibodies of the invention (bio-intact PTH).

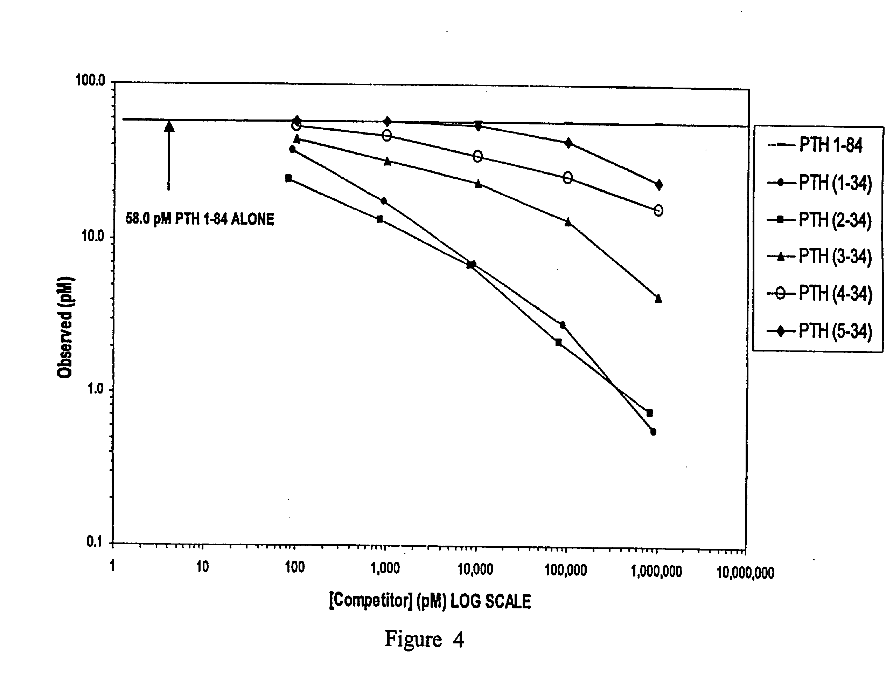

FIG. 4 is a graph of observed PTH concentration (picomoles) as a function of "competitor" concentration (picomoles). The competitors are PTH peptides consisting of amino acids 1-34, 2-34, 3-34, 4-34, and 5-34 of PTH.

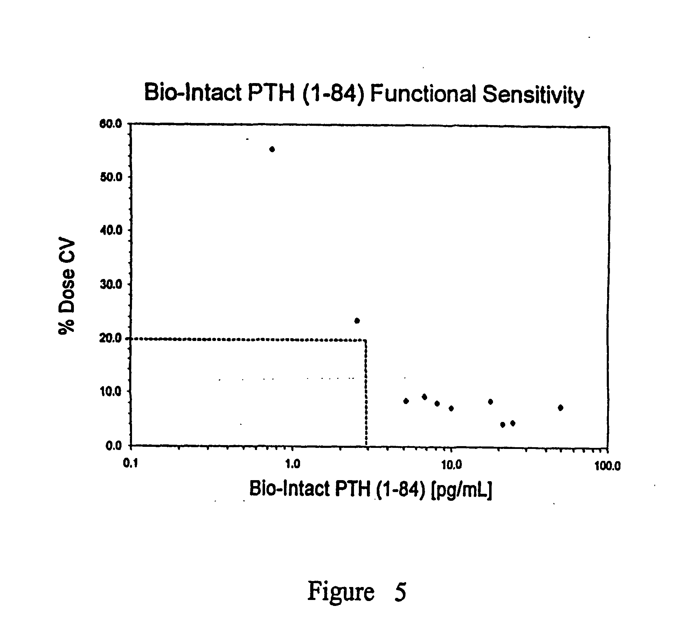

FIG. 5 is a graph of percent dose coefficient of variation versus bio-intact PTH concentration (picograms/mL).

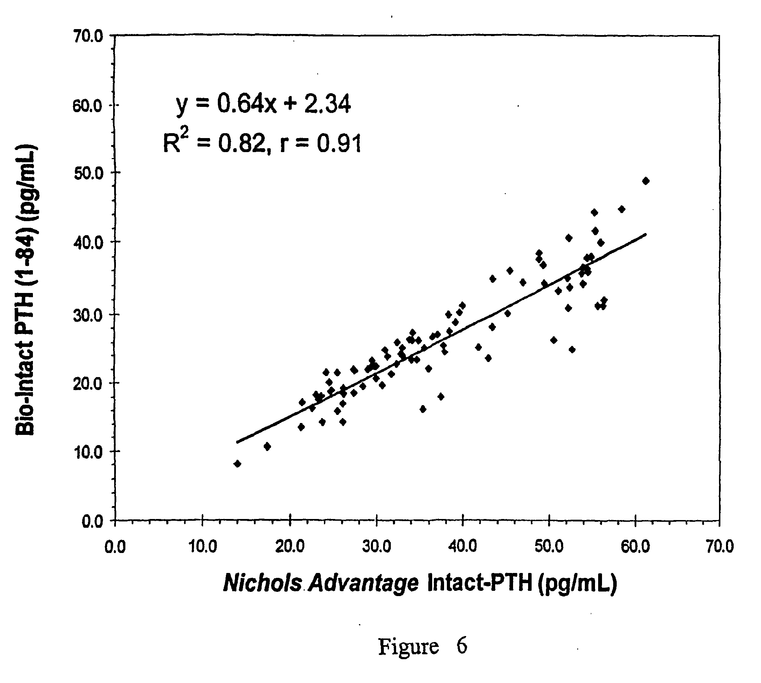

FIG. 6 is a graph of bio-intact PTH concentration (picograms/mL) versus Nichols Advantage intact-PTH concentration (picograms/mL) for "normal" subjects.

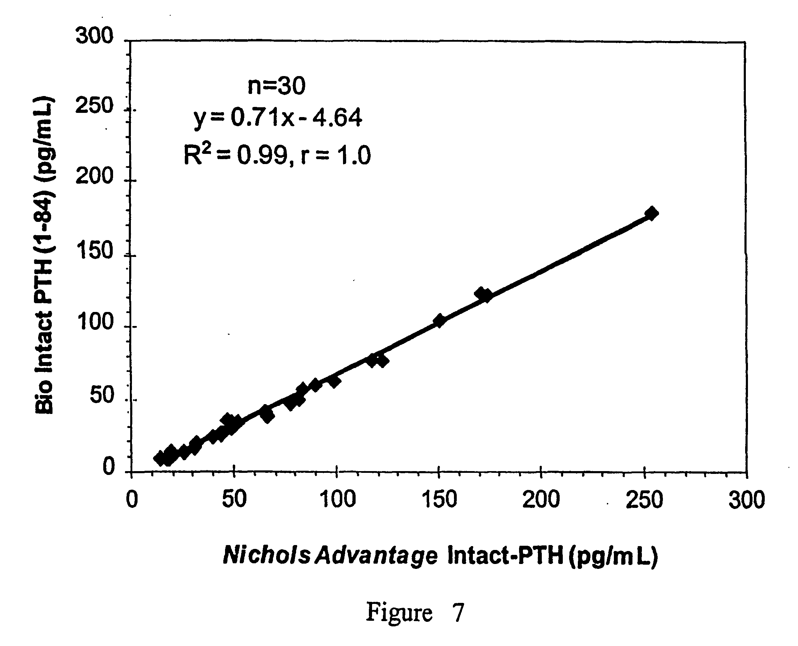

FIG. 7 is a graph of bio-intact PTH concentration (picograms/mL) versus Nichols Advantage intact-PTH concentration (picograms/mL) for subjects with impaired renal function.

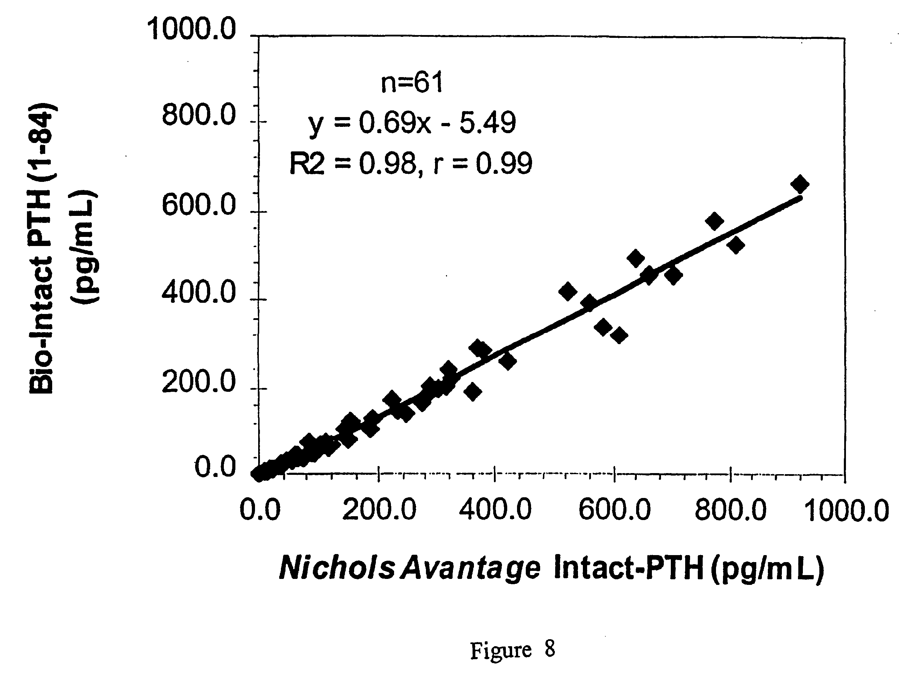

FIG. 8 is a graph of bio-intact PTH concentration (picograms/mL) versus Nichols Advantage intact-PTH concentration (picograms/mL) for subjects with chronic renal failure.

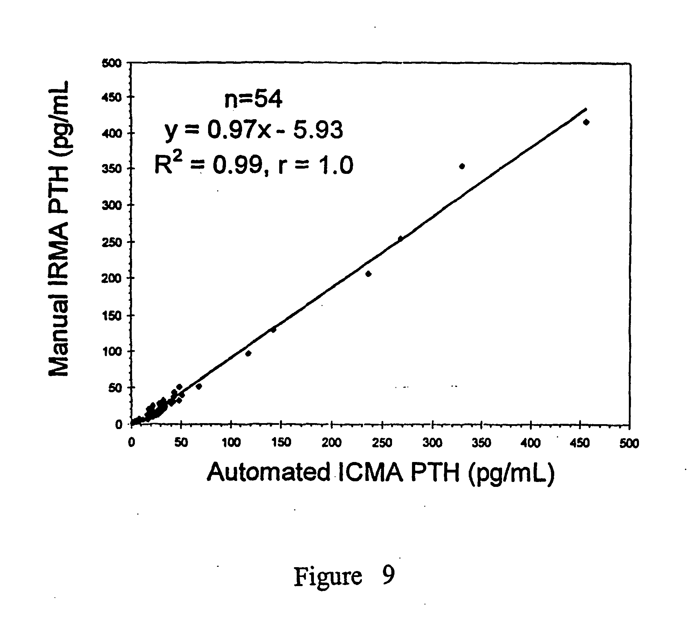

FIG. 9 is a graph of manual immunoradiometric assay PTH concentration (picograms/mL) versus automated ICMA PTH concentration (picograms/mL) for bio-intact PTH. ICMA is defined as Immuno Chemiluminescence Metric Assay. Bio-intact PTH refers to the assay of the invention in which PTH is measured with the antibodies of the invention (e.g., anti-PTH₁₋₁₃) as the detection (tag) antibody, and with anti-PTH₃₈₋₈₄ as the capture antibody.

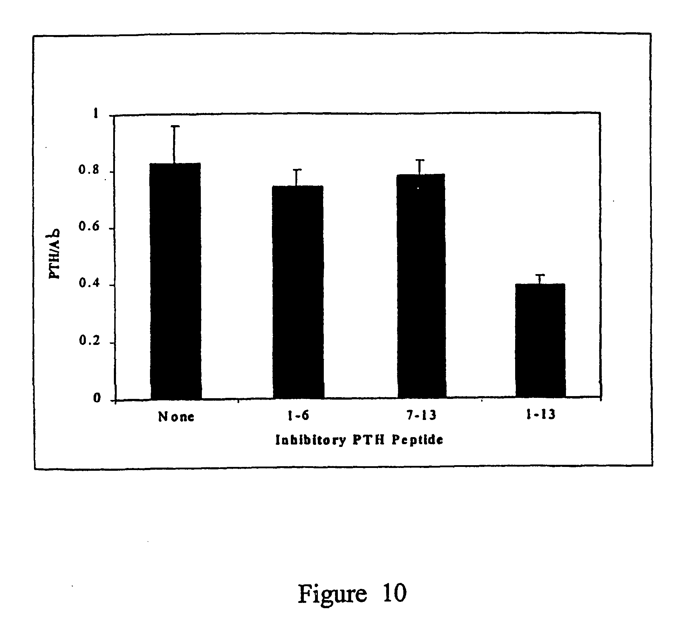

FIG. 10 is a graph illustrating the ratio of PTH to anti-PTH₁₋₁₃ antibodies as a function of the inhibitory PTH peptide. "1-6" indicates the use of a peptide consisting of amino acids 1-6 of PTH (SEQ ID NO: 1); "7-13" indicates the use of a peptide consisting of amino acids 7-13 of PTH (SEQ ID NO: 1); and "1-13" indicates the use of a peptide consisting of amino acids 1-13 of PTH (SEQ ID NO: 1).

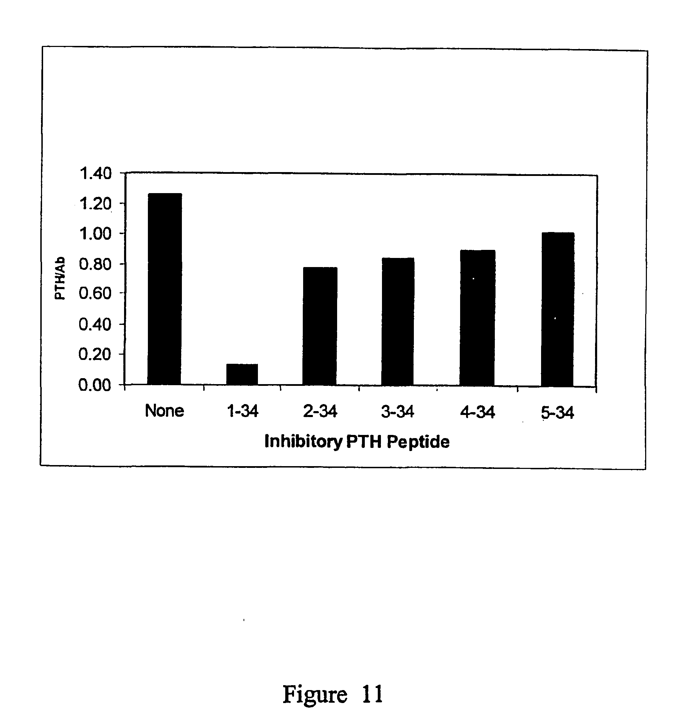

FIG. 11 is a graph illustrating the ratio of PTH to anti-PTH₁₋₁₃ antibodies as a function of the inhibitory PTH peptide. "1-34" indicates the use of a peptide consisting of amino acids 1-34 of PTH (SEQ ID NO: 1); "2-34" indicates the use of a peptide consisting of amino acids 2-34 of PTH (SEQ ID NO: 1); "3-34" indicates the use of a peptide consisting of amino acids 3-34 of PTH (SEQ ID NO: 1); "4-34" indicates the use of a peptide consisting of amino acids 4-34 of PTH (SEQ ID NO: 1); and "5-34" indicates the use of a peptide consisting of amino acids 5-34 of PTH (SEQ ID NO: 1).

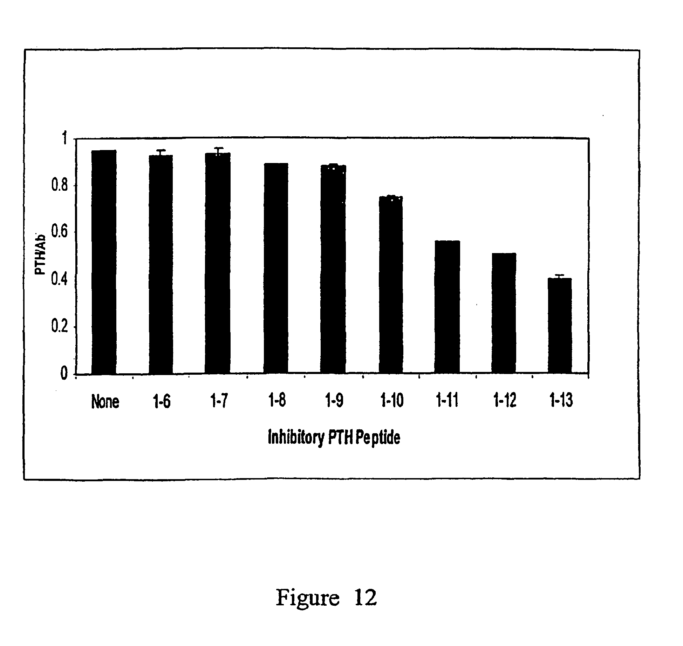

FIG. 12 is a graph illustrating the ratio of PTH to anti-PTH₁₋₁₃ antibodies as a function of the inhibitory PTH peptide. "1-6" indicates the use of a peptide consisting of amino acids 1-6 of PTH (SEQ ID NO: 1); "1-7" indicates the use of a peptide consisting of amino acids 1-7 of PTH (SEQ ID NO: 1); "1-8" indicates the use of a peptide consisting of amino acids 1-8 of PTH (SEQ ID NO: 1); "1-9" indicates the use of a peptide consisting of amino acids 1-9 of PTH (SEQ ID NO: 1); "1-10" indicates the use of a peptide consisting of amino acids 1-10 of PTH (SEQ ID NO: 1); "1-11" indicates the use of a peptide consisting of amino acids 1-11 of PTH (SEQ ID NO: 1); "1-12" indicates the use of a peptide consisting of amino acids 1-12 of PTH (SEQ ID NO: 1); and "1-13" indicates the use of a peptide consisting of amino acids 1-13 of PTH (SEQ ID NO: 1).

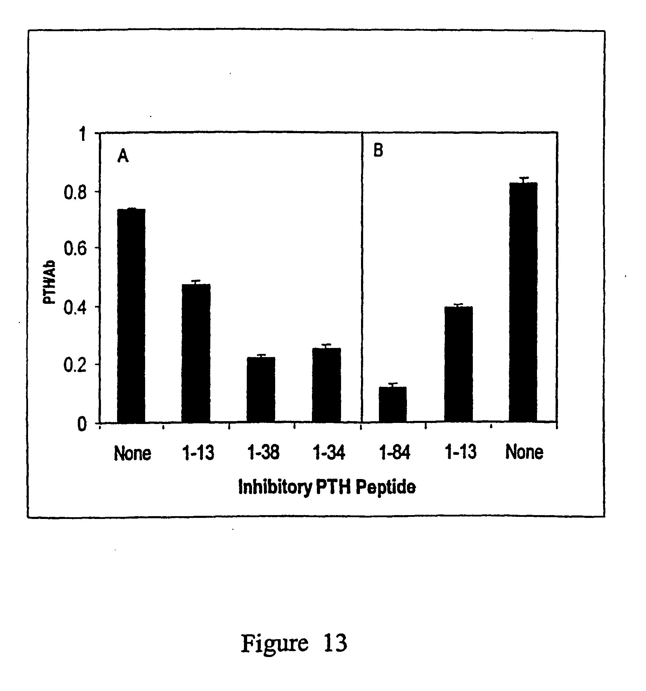

FIG. 13 is a graph illustrating the ratio of PTH to anti-PTH₁₋₁₃ antibodies as a function of the inhibitory PTH peptide. "1-13" indicates the use of a peptide consisting of amino acids 1-13 of PTH (SEQ ID NO: 1); "1-38" indicates the use of a peptide consisting of amino acids 1-38 of PTH (SEQ ID NO: 1); "1-34" indicates the use of a peptide consisting of amino acids 1-34 of PTH (SEQ ID NO: 1); "1-84" indicates the use of a peptide consisting of amino acids 1-84 of PTH (SEQ ID NO: 1); and "1-13" indicates the use of a peptide consisting of amino acids 1-13 of PTH (SEQ ID NO: 1).

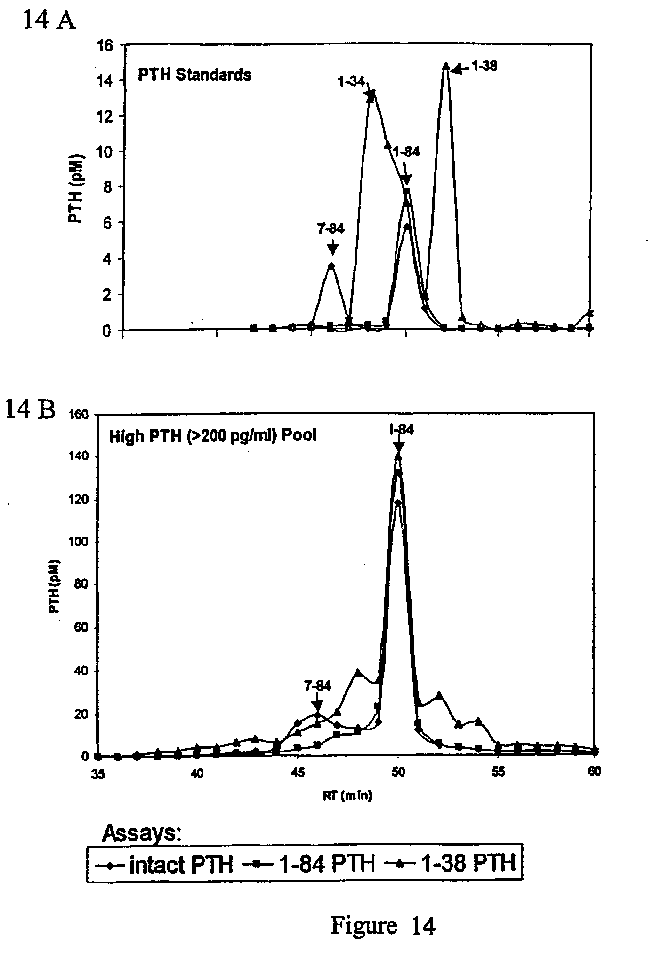

FIG. 14 depicts graphs of PTH concentration (picomoles) as a function of retention time (minutes). FIG. 14A depicts the HPLC measurements for PTH standards (PTH₁₋₈₄; PTH₇₋₈₄; PTH₁₋₃₈; and PTH₁₋₃₄). FIG. 14B depicts the HPLC measurements for a. "high" PTH group (e.g., greater than 200pg/ml). Data are shown for "intact PTH" (closed diamonds); PTH₁₋₈₄ (closed squares); and PTH₁₋₃₈ (triangles).

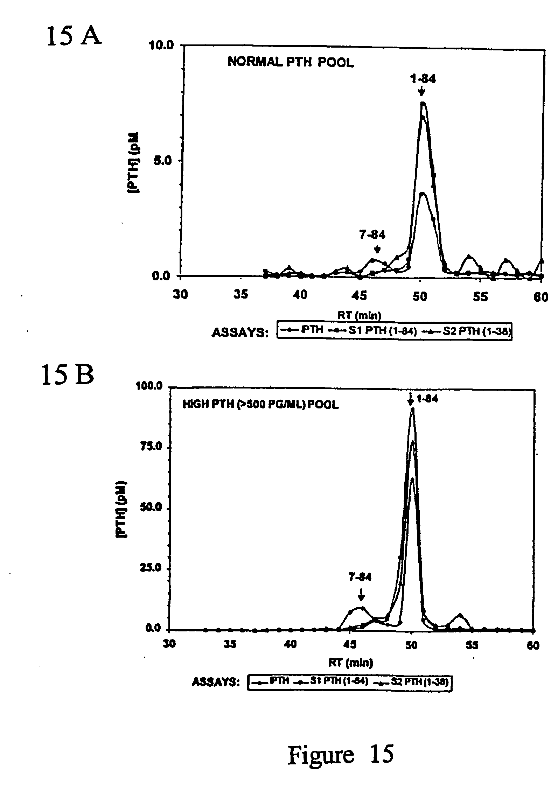

FIG. 15 depicts graphs of PTH concentration (picomoles) as a function of retention time (minutes). FIG. 15A depicts the HPLC measurements for a "normal" PTH group. Data are shown for "intact PTH" (closed diamonds); PTH₁₋₈₄ (closed squares); and PTH₁₋₃₈ (triangles). FIG. 15B depicts the HPLC measurements for a "high" PTH group (e.g., greater than 500pg/ml). Data are shown for "intact PTH" (closed diamonds); PTH₁₋₈₄ (closed squares); and PTH₁₋₃₈ (triangles).

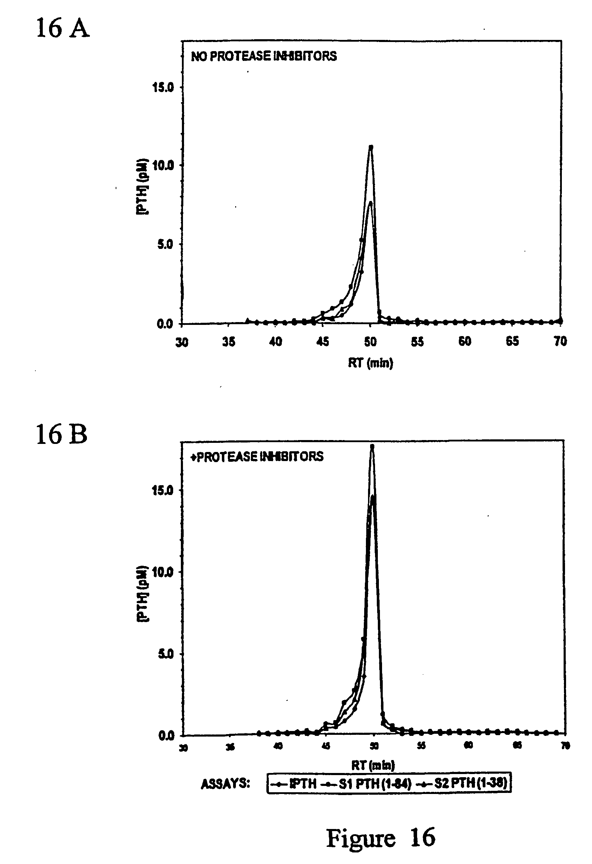

FIG. 16 depicts graphs of PTH concentration (picomoles) as a function of retention time (minutes). FIG. 16A depicts the HPLC measurements for samples without protease inhibitors. FIG. 16B depicts the HPLC measurements for samples with protease inhibitors. Data are shown for "intact PTH" (closed diamonds); PTH₁₋₈₄ (closed squares); and PTH₁₋₃₈ (triangles).

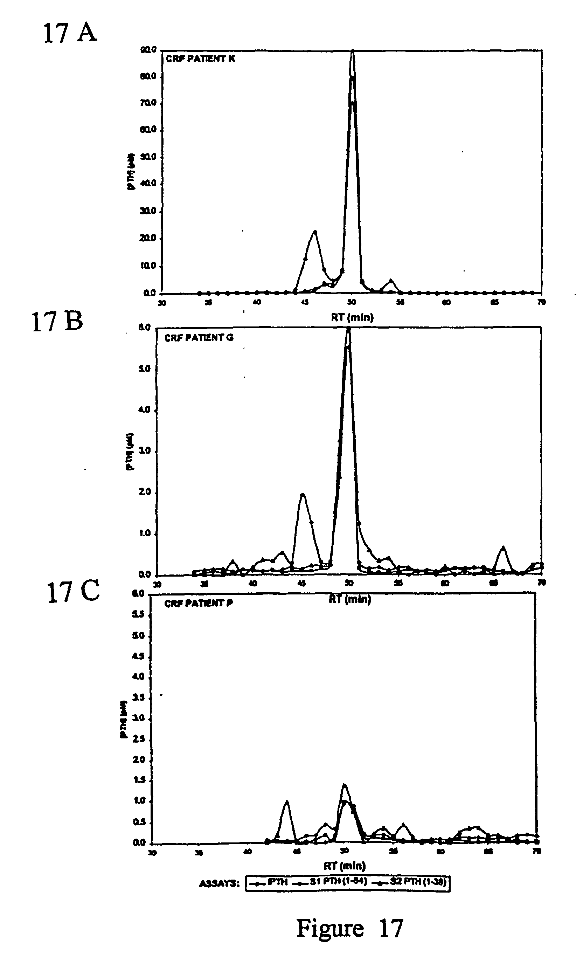

FIG. 17 depicts graphs of PTH concentration (picomoles) as a function of retention time (minutes). FIG. 17A depicts the HPLC measurements for subject K. FIG. 17B depicts the HPLC measurements for subject G. FIG. 17C depicts the HPLC measurements for subject P. Subjects K, G, and P all had chronic renal failure

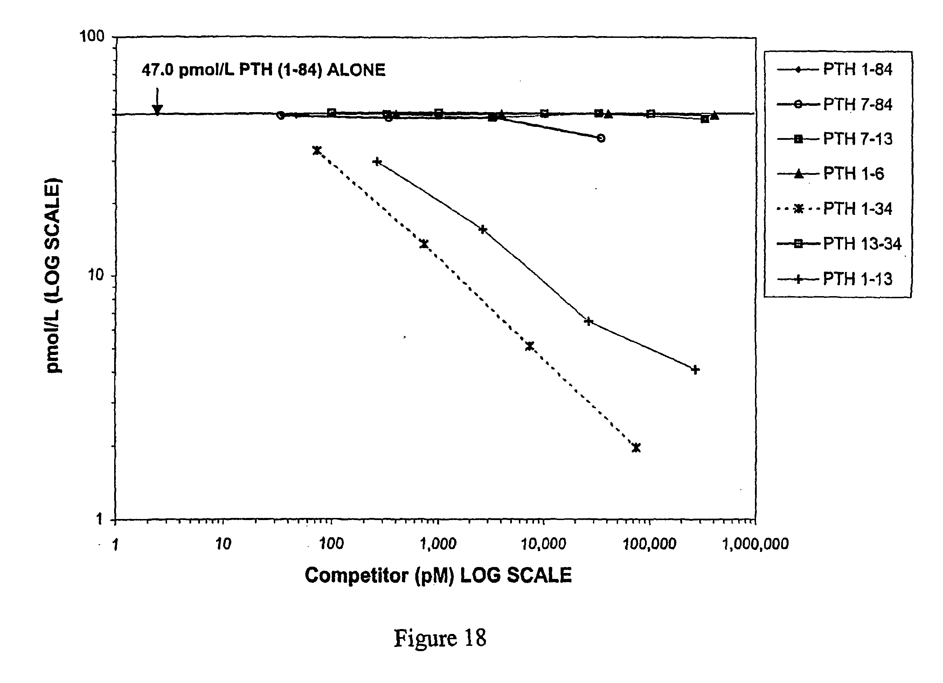

FIG. 18 is a graph of observed PTH concentration (picomoles) as a function of "competitor" concentration (picomoles). The competitors are PTH peptides consisting of amino acids 1-84, 7-84, 7-13, 1-6, 1-34, 13-34, and 1-13 of PTH.

### DETAILED DESCRIPTION OF THE INVENTION

### I. Definitions

Unless defined otherwise, all technical and scientific terms used herein have the same meaning as commonly understood by those of ordinary skill in the art to which this invention belongs. For purposes of the present invention, the following terms are defined below.

As used herein, the terms "PTH" and "parathyroid hormone" are used interchangeably. Parathyroid hormone regulates calcium absorption directly in the kidney and bone, and indirectly in the intestine. Mature parathyroid hormone is synthesized in vivo by cleavage of the signal sequence of "preproPTH", and the subsequent cleavage of the "pro" sequence from "proPTH". Parathyroid hormone, as used herein, encompasses parathyroid hormone from any animal capable of making parathyroid hormone, including, but not limited to, humans, non-human primates, horses, dogs, cats, goats, and rodents. In addition, parathyroid hormone encompasses parathyroid hormone variants, discussed herein, that include recombinantly synthesized parathyroid hormone expressed in organisms that naturally do not express parathyroid hormone.

As used herein, the terms "hPTH", "human PTH", and "human parathyroid hormone" are used interchangeably. In addition, hPTH is encompassed by the terms PTH or parathyroid hormone. Human PTH consists essentially of 84 alpha-amino acid residues arranged by amide linkage in the sequence (SEQ ID NO:1) identified below:

Human PTH can be synthesized in vivo, or synthetically using standard techniques known in the art.

Persons skilled in the art will appreciate that variations in the structure or sequence of PTH may occur. As used herein, a "variant" of PTH is defined as a polypeptide that possesses biological activity that is similar, or substantially similar, to a biological activity of PTH. A molecule is said to be "substantially similar" to another molecule if both molecules have substantially similar structures, or if both molecules possess a similar biological activity. For example, a biological activity of PTH includes the binding of PTH to a receptor for PTH and preventing the subsequent binding or action of PTH at that receptor. Another biological activity of PTH includes regulation of adenylate cyclase activity. Variants of PTH include analogs, fragments, or extensions, of PTH. Variants of PTH include naturally occurring PTH and recombinantly synthesized PTH. For example, a "variant" of PTH may have one or more amino acid substitutions. An amino acid substitution may be conservative or non-conservative, as is well understood in the art. A conservative substitution refers to a substitution of one amino acid with another amino acid without affecting the biological activity, or tertiary (e.g., three-dimensional) structure, of the protein. In addition, variants of PTH include PTH molecules that have modified amino acid side chains, as is well known in the art. Thus, if a molecule possesses at least one biological activity that is similar to an activity of PTH, it is considered a "variant" of PTH. For example, variants include peptides or polypeptides that essentially have the sequence of PTH amino acids in the 1-13 region. Variants also include such peptides with approximately 1 or 2 amino acid substitutions. Further examples of variants of hPTH conist or comprise the following sequences (SEQ ID NOs: 2-8):

As used herein, "bioactive PTH" or "bioactive hPTH" refers to a PTH, or hPTH, polypeptide that has at least one biological activity of PTH. For example, PTH is considered to be bioactive when the polypeptide is able to regulate adenylate cyclase activity. In one embodiment, PTH regulates adenylate cyclase activity by binding to its receptor. Other biological activities are also used to define bioactive PTH. Examples of bioactive hPTH include PTH variants that comprise at least amino acids 1-13 of SEQ ID NO:1. A further example of bioactive PTH includes amino acids 1-34 of SEQ ID NO: 1. A full length PTH molecule, such as amino acids 1-84 of SEQ ID NO: 1, is also a bioactive PTH.

As used herein, "antibody" refers to a polypeptide substantially encoded by an immunoglobulin gene or immunoglobulin genes, or fragments thereof, which specifically recognize and bind an antigen. The recognized immunoglobulin genes include the kappa, lambda, alpha, gamma, delta, epsilon and mu constant region genes, as well as the immunoglobulin variable region genes. Antibodies include fragments, such as Fab', F(ab)₂, Fabc, and Fv fragments. The term "antibody," as used herein, also includes antibody fragments either produced by the modification of whole antibodies or those synthesized de novo using recombinant DNA methodologies, and further includes "humanized" antibodies made by now conventional techniques.

An antibody "specifically binds to" or "is immunoreactive with" a protein when the antibody functions in a binding reaction with the protein. The binding of the antibody to the protein permits determination of the presence of the protein in a sample in the presence of a heterogeneous population of proteins and other agents. Thus, under designated immunoassay conditions, the specified antibodies bind preferentially to a particular protein and do not significantly bind to other proteins present in the sample. Specific binding to a protein under such conditions requires an antibody that is selected for specificity for a particular protein. Several methods for determining whether or not a peptide is immunoreactive with an antibody are known in the art. Immuno chemiluminescence metric assays (ICMA), enzyme-linked immunosorbent assays (ELISA) and radioimmunoassays (RIA) are some examples.

An antibody is "selective for" bioactive PTH when the antibody preferentially binds to bioactive forms of PTH compared to non-bioactive forms of PTH. In a preferred embodiment, an antibody is selective for bioactive PTH when it binds to bioactive PTH at least 10 times more preferentially than non-bioactive PTH. In a more preferred embodiment, the antibody selective for bioactive PTH binds at least 100 times more preferentially than non-bioactive PTH. In another preferred embodiment, the antibody selective for bioactive PTH binds at least 200 times more preferentially than non-bioactive PTH. In yet another preferred embodiment, the antibody selective for bioactive PTH binds at least 500 times more preferentially than non-bioactive PTH. In an even more preferred embodiment, the antibody selective for bioactive PTH binds at least 1000 times more preferentially than non-bioactive PTH.

A "label" is a composition detectable by spectroscopic, photochemical, biochemical, immunochemical, or chemical means. For example, useful labels include fluorescent dyes, chemiluminescent compounds, radioisotopes, electron-dense reagents, enzymes, colored particles, biotin, or dioxigenin. A label often generates a measurable signal, such as radioactivity, fluorescent light, color, or enzyme activity, which can be used to quantitate the amount of bound label.

Examples of chemiluminescent compounds include luciferin, a luminol derivative, pyrogallol, isoluminol, aequorin, cyclic arylhydrazides, dioxetanes, rhodium chelates (electrochemiluminescent), oxalate esters, thermochemiluminescent labels, acridinium and the like. These labels may be attached to a protein, for example an anti-PTH antibody, using techniques well known in the art. (See U.S. Patent No. 5,284,952, ) In one embodiment, a detection antibody, such as an anti-hPTH₁₋₁₃ antibody, may be labeled with an acridinium by employing the methods found in U.S. Patent Nos. 5,284,952, 5,110,932, and 5,338,847.

Examples of the fluorescent material to be used for labeling include fluorescein, fluorescamine, fluorescein isothiocyanate, umbelliferone, rhodamine, Texas red dyes, pthalocyanines, coumarin, squaraine, anthracene, erythrosine, europium chelates and the like.

Examples of radioactive isotopes to be used for labeling include ¹⁴C, ³H, ³²P, ¹⁸F or ¹²⁵I.

Exemplary enzymes which have been developed and can be used in assays of the invention are those described in U.S. Pat. Nos. 3,654,090; 3,791,932; 3,839,153; 3,850,752; U.S. Pat. No. 3,817,837; 3,879,262; Journal of Immunological Methods 1: 247(1972); and the Journal of Immunology 109:129(1972). Other examples of enzymes include, but are not limited to, alkaline phosphatase, beta galactosidase, horseradish peroxidase, gluconidase, phosphatase, peptidase, alkaline phosphatase and the like. Co-enzymes useful in this invention include molecules and/or proteins which facilitate an enzyme to catalyze a reactant to produce a detectable product, for example light. A co-enzyme may include, without limitation, FAD and NAD. In one embodiment, an anti-PTH may be labeled with a NAD. See for example, U.S. Patent 4,380,580.

Examples of colored particles include colloidal gold, or blue latex. In certain embodiments, labels are coupled to the antibodies of the invention to permit determination of the presence of bioactive forms of parathyroid hormone.

Other labels may include a non-active precursor of a spectrophotometrically-active substance (British Pat. No. 1,392,403 and French Pat. No. 2,201,299, which patents correspond to U.S. Pat. No. 3,880,934) and electron spin resonance moieties (U.S. Pat. No. 3,850,578).

The terms "isolated", "purified", or "biologically pure" refer to material that is at least partially separated from, and that is often substantially, or essentially, free from components which normally accompany it as found in its native state. Purity and homogeneity are typically determined using analytical chemistry techniques such as polyacrylamide gel electrophoresis or high performance liquid chromatography, as is well understood in the art. Generally, an isolated antibody will comprise more than 80% of all macromolecular species present in the preparation. Preferably, the antibody is purified to represent greater than 90% of all macromolecular species present. More preferably, the antibody is purified to greater than 95%, and even more preferably, the antibody is purified to essential homogeneity, wherein other macromolecular species are not detected by conventional techniques.

The term "immunoassay" is an assay that utilizes an antibody to specifically bind an analyte. The immunoassay is characterized by the use of specific binding properties of a particular antibody to isolate, target, and/or quantify the analyte.

The inventor of the present invention conducted numerous studies for the purpose of rapidly, readily, and precisely measuring bioactive PTH, specifically hPTH. As a result, he produced an antibody that specifically recognizes bioactive forms of hPTH. The inventor has constructed immunoassays using combinations of antibodies, each recognizing different epitopes of the hPTH.

### II. Compositions of the invention

The antibodies of the invention recognize and bind a three-dimensional epitope of PTH comprising amino acids located between amino acids 1-13 of SEQ ID NO:1 and including the first N-terminal amino acid of native PTH along with the intact helix of the amino terminus. One example of a protein that binds to the antibodies of the invention is human PTH.

The antibodies of the invention have been deposited with the American Type Culture Collection (10801 University Blvd., Manassas, VA 20110-2209, USA) on July 3, 2001, and have been designated Accession No. PTA-3496. The deposit was viable at the time of deposit and will be replaced by Applicants should they become non-viable.

The deposit will be made available to the Commissioner during pendency of the application under the terms of 37 CFR 1.14 and 35 USC § 122, and will be maintained in the depository for a period of 30 years or 5 years after the last request, whichever is longer.

The antibodies of the invention recognize and bind bioactive forms of hPTH (bioactive hPTH). Without wishing to limit the invention to any theory or mechanism of operation, it is believed that the first amino acid (Ser at position 1 of SEQ ID NO: 1) along with the intact helix structure of the amino terminus (N-terminal region) of hPTH must be present in order for hPTH to be bioactive. Furthermore, the bioactive N-terminal region of hPTH is thought to be a three dimensional helical structure consisting of thirteen amino acids (amino acids 1-13 of SEQ ID NO: 1). The antibodies of the invention recognize and bind to a three-dimensional epitope of the N-terminal region. In one embodiment, the epitope comprises all of the thirteen amino acids of hPTH (amino acids 1-13 of SEQ ID NO: 1). In other embodiments, the epitopes comprise a combination of the amino acids within the first thirteen amino acids of hPTH. For example, the epitopes may comprise a combination of amino acids 3, 4, and 5; 2, 4, and 6; 8, 10, and 13; or mixtures thereof.

In one embodiment, antibodies of the present invention recognize and bind at least one amino acid within the first thirteen amino acids and at least one additional amino acid located within the region consisting of amino acids 14 to 84, preferably 13 to 34. For example, antibodies of the present invention may recognize and bind to an epitope comprising amino acids 3, 7, and 14; 4, 8, and 33; or 5, 14, and 15.

In one embodiment of the invention, the antibodies of the invention are polyclonal antibodies.

In another embodiment, the antibodies of the invention are monoclonal antibodies.

In one embodiment of the invention, the antibody recognizes and binds an epitope within the first thirteen amino acids of PTH, or hPTH, and does not bind an epitope of PTH, or hPTH, beyond the first thirteen amino acids. In a preferred embodiment, the antibody recognizes and binds an epitope within the first seven amino acids of PTH, or hPTH.

### III. Methods of making the compositions of the invention

Parathyroid hormone or variants thereof, may be used to generate antibodies by any conventional method well known to those skilled in the art, including those which generate polyclonal antibodies and those which generate monoclonal antibodies.

The polyclonal antibodies of the invention may be produced by immunizing animals with intact PTH, variants thereof, or mixtures thereof, in an emulsion, such as Freund's complete adjuvant. Examples of animals used to produce antibodies include, but are not limited to, mice, rats, rabbits, or goats. Intact PTH may be defined as the full length PTH molecule, for example, the PTH polypeptide after the "pre" and "pro" sequences have been cleaved. In embodiments where human PTH is employed, the intact hPTH has the amino acid sequence depicted in SEQ ID NO: 1. In a preferred embodiment, the intact PTH is preferably linked to a carrier protein, such as keyhole limpet hemocyanin (KLH). Other examples of carrier proteins include bovine serum albumin (BSA), hemocyanin, and bovine thioglobulin (BTG). In certain embodiments, the carrier protein is linked to the antigen of interest (e.g., PTH) with glutaraldehyde, as described herein. Other methods are well known in the art.

After the initial immunization, the animals receive one or more additional immunization boosts of substantially pure, intact bioactive PTH, variants thereof, or mixtures thereof. The immunizations can be administered intraperitoneally, subcutaneously, or intravenously. Thus, the antibodies to surface conformationally correct epitopes are elevated above the large spectrum of antibodies to internal epitopes of PTH.

The animal's immune response to the immunogen preparation may be monitored by taking test bleeds and determining the titer of reactivity to the polypeptide of interest. When approximately high titers of antibody to the immunogen are obtained, blood is collected from the animal and antisera are recovered.

In one embodiment, the antibodies are isolated by exposing the sera to antibody affinity purification columns. Other techniques of isolating the antibodies are well known in the art. The affinity columns are constructed by linking peptides of PTH, preferably hPTH, to a solid phase of the columns. In preferred embodiments of the invention, the columns include hPTH peptides consisting of amino acids 1-13 (hPTH₁₋₁₃), 13-34 (hPTH₁₃₋₃₄), and 39-84 (hPTH₃₉₋₈₄) of hPTH. Other peptides, or combinations thereof, could be utilized to isolate the antibodies of the invention.

As persons skilled in the art will readily understand, the peptides hPTH₁₋₁₃ will specifically bind to antibodies that recognize epitopes of hPTH within the first thirteen amino acids of hPTH. Similarly, peptides hPTH₁₃₋₃₄ will specifically bind to antibodies that recognize epitopes of hPTH within the amino acids 13-34 of hPTH. Also, peptides hPTH₃₉₋₈₄ will bind to antibodies that recognize epitopes of hPTH within the amino acids 39-84 of hPTH.

Thus, by way of the foregoing method, the antibodies that specifically bind to an epitope within the first thirteen amino acids of hPTH (anti-hPTH₁₋₁₃ antibodies) are selectively isolated. In addition, the epitopes recognized by the anti-hPTH₁₋₁₃ antibodies can include any combination of amino acids within the first thirteen amino acids. In one embodiment, the antibodies recognize an epitope consisting of the entire thirteen amino acids of hPTH (i.e., amino acids 1-13 of SEQ ID NO: 1).

As persons skilled in the art will understand, the order in which the antibody-containing sera are exposed to the peptide-coupled affinity columns is not determinative in practicing the invention. However, in preferred embodiments, it is desired to first expose the sera to a column containing the hPTH₃₉₋₈₄ peptides, then to expose the resulting sera (e.g., the sera without the antibodies that bind hPTH₃₉₋₈₄) to a column containing hPTH₁₃₋₃₄ peptides, and subsequently exposing the resulting sera (e.g., the sera without hPTH₃₉₋₈₄ and hPTH₁₃₋₃₄ antibodies) to a column containing hPTH₁₋₁₃ peptides.

Monoclonal antibodies that recognize and bind bioactive PTH are also provided. Monoclonal antibodies can be produced using conventional methods known in the art. See, for example, Kohler and Milstein,(1975) Nature, 256:495-97. Briefly, animals, such as mice, are injected with PTH preferably coupled to a carrier protein as described above. The animals are boosted with one or more pure PTH injections, and are hyperimmunized by an intravenous (IV) booster 3 days before fusion. Spleen cells from the mice are isolated and are fused by standard methods to myeloma cells. Hybridomas are selected in standard hypoxanthine/aminopterin/thymine (HAT) medium, according to standard methods. Hybridomas secreting antibodies which recognize bioactive PTH are identified, cultured, and subcloned using standard immunological techniques.

Frequently, the antibodies will be labeled by joining, either covalently or non-covalently, a substance which provides for a detectable signal. In one embodiment of the invention, the labels are chemiluminescent compounds. A preferred chemiluminescent compound is an acridinium ester.

### III. Methods of using the composition of the invention

### A. Immunoassays

The immunological assays of the present invention can be used for quantifying an antigen in a sample. Examples of various assays and related reagents and parameters are provided in U.S. Ser. No. 09/761,969, filed January 16, 2001.

One example of an immunoassay is a "sandwich" immunoassay. In the sandwich immunoassay, an antibody is immobilized on a solid phase (capture antibody), incubated with an antigen, and further incubated with an antibody with a detectable label (detection antibody). In a preferred embodiment of the invention, a PTH antibody that does not recognize the bioactive portion of PTH is coupled to a solid phase, such as magnetic particles, the antibody is incubated with a sample containing PTH, and is further incubated with an antibody that specifically binds bioactive PTH, coupled to a detectable label. In a more preferred embodiment, a hPTH antibody that does not recognize amino acids 1-13 of hPTH is coupled to magnetic particles, the antibody is incubated with a sample containing hPTH, and is further incubated with an antibody that recognizes bioactive hPTH coupled to an acridinium ester. In the sandwich immunoassay of the present invention, a sample, the first antibody and the labeled second antibody may be incubated simultaneously. Examples of suitable solid phase substrates include, but are not limited to, microtiter plates, beads, tubes, membranes, filter papers, or plastic cups.

Another example of the immunological assays include assays in which an antigen labeled with a detectable label and an unlabeled antigen are competitively reacted with an antibody. In addition, an antigen can be immobilized on a solid phase, incubated with a diluted antiserum or a purified antibody, and further incubated with an antiimmunoglobulin labeled with a detectable label, thereby obtaining a labeled binding substance.

When the sandwich technique is used, two kinds of antibodies against different epitopes of PTH are first prepared. One antibody is labeled with a detectable label (detection antibody) and the other antibody is allowed to bind to a solid phase as a solid phase antibody or is made to be able to specifically bind to a solid phase (capture antibody). These antibodies are allowed to react with antigens in various concentrations to form a plurality of antigen-antibody complexes. Since the antigen-antibody complexes are bound to solid phases, the solid phases are separated from the complexes, and the amount of label in the solid phases is measured. The relationship between the label and the concentration of the antigen is plotted to obtain a standard curve.

When a sample including an unknown concentration of antigen is added to the reaction system, the concentration of the antigen can be determined by applying the amount of label measured after the reaction to the standard curve. Examples of samples to be measured can be those containing PTH, or variants thereof, and include plasma, serum, blood, urine, and the like.

The sandwich immunoassay of the present invention includes a sandwich radioimmunoassay (RIA), a sandwich enzymeimmunoassay (EIA), a sandwich fluoroimmunoassay (FIA), a sandwich chemiluminescence immunoassay (CLIA, ICMA), a sandwich chemiluminescence-enzymeimmunoassay (CLEIA) and an immunochromatographic method based on the sandwich assay.

The coupling of the capture antibody to the solid phase, and the labeling of the detection antibody can be performed by any method known to those skilled in the art. In a preferred embodiment of the invention, the capture antibody is coupled to biotin, and the magnetic particles are coated with streptavidin. The biotin binds to the streptavidin, thereby achieving the coupling of the capture antibody to the solid phase.

### B. SCREENING ASSAYS

The present invention further provides assays for detecting levels of PTH in a subject. In a preferred embodiment, the assays provide methods for detecting abnormal levels of PTH in a subject's blood.

These assays are particularly useful for screening test compounds to determine their ability to regulate PTH activity. In such cases, a test compound is added to the reaction system and the effect of the test compound on the binding of antibodies to PTH can be observed. Those compounds which inhibit the binding of the antibodies of the invention to bioactive PTH can be considered as potential bioactive PTH inhibitors and further as potential therapeutic agents for treatment of conditions associated with abnormal PTH activity or concentrations.

In one example, those antibodies that recognize PTH are screened for their ability to compete with PTH for binding to a PTH receptor. The antibody used may be from crude antiserum, cell medium, or ascites, or in purified form. Antibodies that reduce binding of the PTH analog to the PTH receptor are classified as competitive; those which do not are noncompetitive.

Compounds, including antibodies, may be screened for their agonistic or antagonistic properties using cAMP accumulation, intracellular calcium, and/or inositol phosphate assays. Cyclic AMP accumulation can be measured by the assays, as described above. A compound that competes with PTH for binding to the PTH receptor, and that inhibits the effect of PTH on cAMP accumulation, is considered a competitive PTH antagonist. Conversely, a compound that does not compete for PTH binding to the PTH receptor, but which still prevents PTH activation of cAMP accumulation (presumably by blocking the receptor activation site) is considered a non-competitive antagonist. A compound that competes with PTH for binding to the PTH receptor, and which stimulates cAMP accumulation in the presence or absence of PTH, is a competitive agonist. A compound that does not compete with PTH for binding to the PTH receptor but which is still capable of stimulating cAMP accumulation in the presence or absence of PTH, or which stimulates a higher accumulation than that observed by PTH alone, would be considered a non-competitive agonist.

### C. Diagnostic uses

The antibodies of the invention are useful for the diagnosis, classification, prognosis, and/or treatment of disorders which may be characterized as related to the interaction between a cell receptor of the invention and its specific ligand. For example, some forms of hypercalcemia and hypocalcemia are related to the interaction between PTH and the PTH receptor(s). Hypercalcemia is a condition in which there is an abnormal elevation in serum calcium level; it is often associated with other diseases, including hyperparathyroidism, osteoporosis, carcinomas of the breast, lung and prostrate, epidermoid cancers of the head and neck of the esophagus, multiple myeloma, and hypernephroma. Hypocalcemia, a condition in which the serum calcium level is abnormally low, may result from a deficiency of effective PTH.

In one example, the compounds of the invention are used to manufacture diagnostic agents which are used as diagnostic tools to diagnose hypercalcemia and to distinguish between-hypercalcemic conditions, i.e., to differentiate hypercalcemia mediated by PTH (e.g., hyperparathyroidism and humoral hypercalcemia of malignancy), from hypercalcemia associated with diseases which do not involve these factors (e.g., local osteolytic hypercalcemia mediated by the presence of metastatic tumor cells in direct contact with bone, and certain rare types of malignancy-related hypercalcemias mediated by an increase of humoral factors, such as osteoclast activating factor (interleukin), lymphotoxin, calcitriol, type E prostaglandins, and vitamin D-like sterols).

In one method of diagnosis, serum total and/or ionized calcium levels are measured by standard techniques before and after the administration of the PTH antagonists of the invention. PTH related hypercalcemias would be detectable as a decrease in serum calcium levels following administration of the antagonist of the invention. In contrast, for hypercalcemic conditions mediated by factors other than PTH, the serum calcium levels would remain unchanged even after administration of the antagonist.

Another diagnostic application of the invention permits measurement of the level of PTH in a biological sample in order to diagnose PTH related tumors, e.g., tumors which are associated with humoral hypercalcemia of malignancy, and for monitoring the levels of PTH during cancer therapy. This method involves assaying binding of the parathyroid hormone receptor to PTH present in a tissue sample, using one of the assays described herein.

A patient who is suspected of being hypercalcemic may be treated using the compounds of the invention. Rapid intervention is important because symptoms may appear abruptly and, unless reversed, can be fatal. In one application, serum calcium levels are stabilized by an immediate course of treatment which includes antagonists of PTH. Such antagonists include the compounds of the invention which have been determined (by the assays described herein) to interfere with the biological activity of PTH. To administer the antagonist, the appropriate antibody (is used in the manufacture of a medicament, generally by being formulated in an appropriate carrier such as physiological saline, and administered intravenously, at a dosage that provides adequate competition for PTH binding to the PTH receptor (e.g., a dosage sufficient to lower the serum calcium level to below 10 mg/dl). Typical dosage would be 1 ng to 10 mg of the antibody per kg body weight per day. Treatment may be repeated as necessary for long term maintenance of acceptable calcium levels (i.e., levels <10.1 mg/dl). In addition, other modes of administration can be used to administer a medicament of the invention. This may be necessary for acute treatment of an underlying disease condition triggering hypercalcemia; or it may used, e.g., for chronic treatment of conditions such as osteoporosis.

### D. Pharmaceutical compositions and therapeutic methods

Further disclosed are pharmaceutical compositions incorporating the compositions of the invention and including a pharmaceutically acceptable carrier. Such pharmaceutical compositions should contain a therapeutic or prophylactic amount of at least one antibody identified by the methods of the present invention. The pharmaceutically acceptable carrier can be any compatible, non-toxic substance suitable to deliver the compounds to an intended host. Sterile water, alcohol, fats, waxes, and inert solids may be used as the carrier. Pharmaceutically acceptable adjuvants, buffering agents, dispersing agents, and the like may also be incorporated into the pharmaceutical compositions. Preparation of pharmaceutical conditions incorporating active agents is well described in the medical and scientific literature. See, for example, Remington's Pharmaceutical Sciences, Mack Publishing Company, Easton, Pa., 16th Ed., 1982.

The pharmaceutical compositions just described are suitable for systemic administration to the host, including both parenteral and oral administration. The pharmaceutical compositions will usually be administered parenterally, i.e. subcutaneously, intramuscularly, or intravenously. Thus, the present invention provides compositions for administration to a host, where the compositions comprise pharmaceutically acceptable solution of the identified compound in an acceptable carrier, as described above.

The concentration of the compound in the pharmaceutical carrier may vary widely, i.e. from less than about 0.1% by weight of the pharmaceutical composition to about 20% by weight, or greater. Typical pharmaceutical composition for intramuscular injection would be made up to contain, for example, one to four ml of sterile buffered water and one µg to one mg of the compound identified by the method of the present invention. The typical composition for intravenous infusion could be made up to contain 100 to 500 ml of sterile Ringer's solution and about 1 to 100 mg of the compound.

Various kits comprising a releasing composition and a detecting composition as disclosed in this invention may be used for determining the concentration of PTH, or variants thereof, in a sample. However, such are only examples of the many possible kits which may employ the compositions of the invention. Other kits are contemplated to be within the scope of this invention. For example, the following patents, articles and instruction manuals disclose assay methods which may be adapted to include a releasing composition of the present invention: U.S. Patent No. 4,935,339: No. 4,121,1978; No. 5,232,836; No. 5,064,770; No. 5,202,266; No. 4,816,417; No. 5,821,020 and No. 5,981,779. One adaptation would be to replace the compositions used therein with the present compositions.

In a broad embodiment, the kit of the present invention may be adapted to be employed in an automated assay system to determine the concentration of PTH or variants thereof. For example, the kit of the present invention may preferably be used in conjunction with the Nichols Advantage system.

### EXAMPLES

### Example 1

Production of antibodies that recognize and bind bioactive hPTH

### A. Immunization

Human Parathyroid Hormone 1-84 was purchased from BACHEM, California Inc., Torrance, CA. Product No. H-1370.1000.

KLH, Hemocyanin, Keyhole Limpet was purchased from Calbiochem, La Jolla, CA. Product No. 374805.

Glutaraldehyde was purchased from Sigma Chemical Co. St. Louis, MO. Product No. G-6247.

Human Parathyroid Hormone was coupled to KLH in molar ratios of 200:1 as follows:

One milligram of PTH was solubilized in 0.20 mL 0.1 N Acetic Acid. The solubilized PTH was diluted to 1 mg/mL in 0.1M Na Phosphate, 0.15 M NaCl pH 7.4. Keyhole Limpet Hemocyanin (KLH) was solubilized in 0.1 M Na Phosphate pH 7.2 to a concentration 10 mg/mL by protein content. Molar ratios by protein content of 200 moles PTH to 1 mole of KLH were calculated and the two proteins mixed at 4 degrees C. A 10 mg/ml (1%) solution of Glutaraldehyde was prepared by diluting the 25% aqueous solution to 1% in 0.1M Na Phosphate pH 7.2. The mixture of 200 moles PTH to 1 mole KLH were coupled by the addition of 1% glutaraldehyde to a final glutaraldehyde concentration of 0.655 mg/mL. The gluataldehyde cross-linked protein mixture was kept at 4 degrees C for 5-8 hours and the reaction stopped by dilution by the addition of an equal volume of 0.01 M Na Phosphate, 0.15 M NaCl pH 7.4. The total protein concentration of the PTH-KLH was calculated on the assumption of an 80% crosslinking efficiency.

Goats, purchased through Strategic BioSolutions, Ramona, CA, were boarded and immunized as follows:

For the first immunization, goats were immunized with 2 ml of 0.1 mg of PTH-KLH solubilized with an equal volume of Complete Freund's Adjuvant. One mL with 0.1 mg PTH-KLH with one mL Complete Freund's Adjuvant. The animals were injected intradermally on the animals at multiple sites.

Subsequent boosts were performed on a 3-4 week bases (monthly) with 2 mL of 0.05 mg of PTH-KLH, prepared as above, in an equal volume of Incomplete Freund's and injected intradermally, as before, on the animal's side. At least two to three boosts of PTH-KLH in Incomplete Freund's were performed.

For all subsequent boosts, the animals were then immunized only with 0.02 mg of pure PTH₁₋₈₄ solubilized, as above, in Incomplete Freund's Adjuvant for 2 mL of immunogen, and injected as before on the animal's side intradermally. In this manner, antibody clones to conformation epitopes are boosted preferentially to internalize linear PTH sequences.

After at least 2 boosts of pure PTH₁₋₈₄, the animals were bled, and off-the-clot serum was collected. The specificity and titer were determined using Surface Plasmon Resonance (SPR) using BIAcore instrumentation.

### Example 2

### Isolation of antibodies to conformational PTH₁₋₁₃

Affinity columns to PTH amino acid sequences PTH₃₈₋₈₄, PTH₁₃₋₃₄ and PTH₁₋₁₃ were constructed as follows:

PTH₃₈₋₈₄ and PTH₁₃₋₃₄ were purchased through BACHEM, California, Product numbers H-4926.1000 and H-4145.1000, respectively. PTH₁₋₁₃ was chemically synthesized by Research Genetics, Huntsville, Al.

Each of the respective PTH peptides were coupled to CNBr Activated Sepharose 4-B, by Pharmacia, as outlined in their procedure, with 1-2 mg of peptide to 2 mL of Sepharose 4-B.

Affinity purified antibody to PTH₁₋₁₃ was prepared as follows:

Goat immune serum prepared as above, was sequentially purified on the affinity columns first to remove anti-PTH₃₈₋₈₄ antibodies, then anti-PTH₁₃₋₃₄ antibodies, and finally anti-PTH₁₋₁₃ antibodies. The serum extraction for all three affinity columns is the same:

At least 0.5 liters of goat immune serum was passed of the respective affinity columns in the order noted above. Each affinity column consisting of at least 10 mg of peptide linked to Sepharose-4B.

The columns were washed extensively with 0.01 M Na Phosphate, 0.15 M NaCl pH 7.4. until the Spectrophotometric absorption at 280 nm no longer changed.

To remove non-specific serum proteins, the columns were then washed with 0.1 M Na Acetate, 0.15 M NaCl pH 4.0, again monitoring the absorption at 280 nm. Specific antibodies to the peptide sequences were then eluted with 0.2 M Glycine pH 2.3. The low pH 2.3 elution by the week buffer glycine dissociates the antibody from the covalently linked peptide to Sepharose 4-B. The final eluted antibodies to PTH₁₋₁₃ are then neutralized with the addition of dilute 0.1 N NaOH to bring the pH back to 7.4.

The affinity purified goat anti-PTH₁₋₁₃, is then tested for purity by HPLC analysis. Protein concentration determined by molar extinction coefficient, absorption at 280 nm, 1.4 A²⁸⁰ = 1 mg/mL. The affinity purified antibody binding and specificity then were determined using SPR by BIAcore instrumentation. The antibody is stored at 4 degrees C, or - 70 degrees C for long term storage.

Antibody affinity purification columns consisting of hPTH₁₋₁₃, hPTH₁₃₋₃₄, and hPTH₃₉₋₈₄ are constructed with these peptides respectively liked to Sepharose 4B. The animal serum generated in the immunization sequence can be isolated for these respective antibodies in the following sequence. First antibodies to hPTH₃₉₋₈₄ are removed form the sera, followed by removing antibodies to hPTH₁₋₃₄. Then the final affinity column of hPTH₁₋₁₃ is used to isolate the anti-hPTH₁₋₁₃ antibodies which recognize the bioactive conformationally correct N-Terminal sequence of hPTH. Although in our experiments the antibodies to hPTH₁₃₋₃₄ and hPTH₃₉₋₈₄ were first removed to prove there were no overlapping epitopes, it is possible to isolate the bioactive hPTH₁₋₁₃ conformational antibodies without removing the other antibodies first.

### EXAMPLE 3

### Labeling of anti-PTH₁₋₁₃ antibody with acridinium

A purified anti-PTH₁₋₁₃ antibody was labeled with acridinium by the method as follows:

Acridinium as a "sulfonyl chloride ester" is crosslinked to the antibody of the invention by the reaction of the lysly moiety of the epsilon amino group of lysine in proteins, such as antibodies, to the acridinium ester. The reaction products are separated by size exclusion chromatography on Sepharose G-25 with 0.1 M Na Phosphate, pH 6.0.

### EXAMPLE 4

### Immunometric assay for bioactive hPTH

Affinity purified antibodies to bioactive N-Terminal PTH (anti-PTH₁₋₁₃) are labeled for detection and anti-PTH₃₉₋₈₄ antibodies are labeled for capture.

Biotinylated capture antibodies (anti-PTH₃₉₋₈₄) are used to bind PTH from a sample. The capture antibodies recognize both full length PTH (PTH₁₋₈₄) and C-terminal fragments of PTH. Acridinium labeled detection antibodies (anti-PTH₁₋₁₃) are used to bind the bioactive N-terminal epitope of PTH in a sample. The capture antibody preferably recognizes and binds bioactive PTH (e.g., PTH that has a conformationally correct N-terminal region). The capture and detection antibodies "sandwich" the PTH molecules in the sample. The sandwich complex is bound to streptavidin-coated magnetic particles. The particles are then quantitated in a luminometer as is known in the art. The procedures are illustrated in FIG. 1.

Samples are obtained from normal subjects (e.g., subjects that are apparently healthy, ambulatory, and non-medicated. The normal subjects have normal calcium and 25(OH) vitamin D levels. Samples are also obtained from sera of subjects that was submitted for creatine clearance, and had a creatine clearance of less than 70 mL/min. Samples were also obtained from subjects experiencing chronic renal failure (CRF). These samples consisted of EDTA plasma obtained from subjects on dialysis.

In a preferred embodiment of the invention, 150 µL of the biological samples were combined with 70 µL of biotinylated goat anti-PTH₃₉₋₈₄ capture antibodies, 25 µL acridinium labeled goat anti-PTH₁₋₁₃ antibodies, and 15 µL streptavidin coated magnetic particles. The combination was incubated at 37 degrees C for 30 minutes. The combination was washed, and read in a luminometer.

Using an immunoradiometric assay (IRMA) for PTH₁₋₈₄, where the anti-PTH₁₋₁₃ is labeled for detection and anti-PTH₃₉₋₈₄ is labeled for capture, the following observations are noted. Fragments, PTH₁₋₆ and PTH₇₋₁₃ will not inhibit the assay. PTH fragments consisting of PTH₁₋₃₄ strongly inhibit the assay, where PTH fragments 2-34, 3-34, 4-34 and 5-34 inhibit the assay in diminishing response as amino acids are removed from amino acid position one on PTH₁₋₃₄ (FIG. 4). Results with other fragments are shown in FIG. 18. Inhibition with a PTH₇₋₈₄ fragment also was observed to have no effect. Indicating that the conformational epitope is strongly dependent on at least full residue sequence in PTH₁₋₁₃.

This assay was then used in a study with clinical patient samples that demonstrate the presence of PTH₇₋₈₄ fragments. The study as shown clearly demonstrates the clinical utility of the bioactive intact N-terminal antibodies in be able to detect only the bioactive intact PTH₁₋₈₄. The correlation studies using the antibodies of the invention and the NID Intact-PTH assay in patients with early and end-stage renal disease suggest the proportion of intact PTH (PTH₁₋₈₄) to PTH fragments (PTH₇₋₈₄) is similar (slopes of 0.71 and 0.69, respectively), but is different from the normal subjects (slope of 0.52). See FIGS. 6-8).

### Example 5

### Surface Plasmon Resonance (SPR)

Surface Plasmon Resonance (SPR) is a technique in which a response is measured by the change in the refractive index of light on a surface as a function of the mass of material bound to the surface. Using a commercial instrument by BIAcore, SPR has been used to measure the kinetics of noncovalent interactions between antibodies and their antigens. See, for example, Malmqvist, M., Curr. Opin. Immunol. 5, 282-286, (1993); O'Shannessy, D.J., Curr. Opin. Biotechnol. 5, 65-71, (1993); Schuck, P., Curr. Opin. Biotechnol. 8, 498-502, (1997); the contents of which in their entireties are incorporated by reference. The BIAcore instrument has been demonstrated to be a unique tool in the measurement of immunoreagents (Adamczyk, M. et al., Bioconjugate Chem. 10, 176-185, (1999); and Adamczyk, M., et al., Bioconjugate Chem. 8, 133-145, (1997); the contents of which in their entireties are incorporated by reference.

In brief, CM5 sensor chips with covalently coupled rabbit anti-goat Fc antibodies were used. The buffer used was phosphate buffered saline (PBS) at a pH of 7.4 with 0.005% surfactant P-20. The flow rate was 10 µL/min. Approximately 500 response units (RU) of the antibodies of the invention was immobilized per run. The method generally comprised a one minute injection of the detection antibody (i.e., anti-PTH₁₋₁₃ antibody) at a rate of 40 µg/mL followed by a two minute injection of an inhibitory peptide (i.e., a PTH fragment, as described herein) at a rate of 100 µg/mL followed by a two minute wash followed by a one minute injection of PTH₁₋₈₄ at one µg/ml. Binding of the antibody and PTH₁₋₈₄ was measured 30 seconds after the end of each injection. After each run, the surface was regenerated with 10 mM gly-HCl for regeneration.

The results of the SPR studies demonstrate the conformational specificity of the N-Terminal anti-hPTH₁₋₁₃ antibodies as follows: In FIG. 10, PTH sequences 1-6 and 7-13 cannot compete for the binding of the anti-hPTH₁₋₁₃ antibodies alone, and are therefore important for antibody binding. PTH₁₋₁₃ does inhibit binding of PTH to the antibodies of the invention. The data demonstrate that the antibodies of the invention bind to amino acid residues in both PTH₁₋₆, and PTH₇₋₁₃ fragments. The intact full three-dimensional epitope consisting of at least 13 amino acids must be present for binding.

FIG. 11 demonstrates that amino acid residues 1 and 2 at the N-terminus are important for binding of the N-terminal anti-PTH antibody. The removal of the first amino significantly reduces the binding of the antibody because the correct conformational structure is lost. In that regard, the peptides PTH₁₋₃₄ and PTH₂₋₃₄ show the greatest inhibition of binding of PTH to the antibodies of the invention.

In FIG. 12, removal of residues 10-13 (especially 13) limits the binding of the N-Terminal Anti-PTH antibody. PTH peptides, PTH₁₋₆, and PTH₁₋₇ do not inhibit binding of PTH₁₋₈₄ to the antibodies of the invention, and that the inhibition of binding becomes successively more pronounced as the size of the inhibitory peptide increases from PTH₁₋₁₀ to PTH₁₋₁₃.

In FIG. 13, N-terminal PTH peptides 1-34, 1-38 and 1-84 inhibit the binding of PTH to the N-terminal antibody more strongly than PTH 1-13 These results indicate that the PTH 1-13 peptide, although conformational, does not fold into exactly the same three dimensional structure that PTH amino acids 1-13 assume when they are part of a larger PTH molecule, further demonstrating the unique conformational binding of bioactive intact N-Terminal PTH 1-13 antibodies.

These results together identify amino acid residues in two distinct regions of PTH (the N-Terminus region amino acids 1-2 and 10-13), which are important determinants for antibody binding. Since these regions are not juxtaposed in the linear sequence of PTH, the bioactive intact N-Terminal Anti-PTH 1-13 antibodies recognize a conformational (nonlinear) epitope of PTH.

### Example 6

### High pressure liquid chromatography (HPLC)

Biological samples are extracted from subjects, and are eluted with 80% acetonitrile in 0.1 % trifuoroacetic acid (TFA) The resulting concentrate sample was dried in a speed vacuum. The dry sample was then solubilized in a minimal volume (e.g., 0.5 mL) of 0.1% TFA.

HPLC was performed on an analytical C18 column, at a flow rate of 1.5 mL/min, with a linear acetonitrile gradient in 0.1% TFA, collecting 1.0 minute fractions. The concentrated fractions were than dried in a speed vacuum. The resulting fractions were subsequently solubilized in 0.7% bovine serum albumin (BSA) in phosphate buffered saline (PBS). The fractions were subsequently assayed on a Nichols Institute Diagnostics (NID) Advantage with Intact PTH, the bio-intact PTH assay (i.e., the assay of the invention), and the PTH₁₋₃₈ assay.

The NID Intact PTH assay recognizes PTH₇₋₈₄, and PTH₁₋₈₄. The bio-intact assay recognizes PTH₁₋₈₄, but does not recognize PTH₇₋₈₄ (FIG. 3). The PTH₁₋₃₈ assay recognizes PTH₁₋₈₄, PTH₁₋₃₄, PTH₁₋₃₈, but does not recognize PTH₇₋₈₄.

The results of the HPLC experiments indicate that HPLC resolves chemically synthesized PTH fragments (PTH₁₋₈₄, PTH₁₋₃₄, PTH₁₋₃₈, and PTH₇₋₈₄) (FIG. 14A). HPLC resolves PTH in patient samples primarily in two major peaks, as detected by the NID assay. The first peak corresponds to PTH₇₋₈₄ fragments, and the second peak corresponds to PTH₁₋₈₄ (FIG. 3, 14B, 15A, and 15B). As persons skilled in the art will appreciate, the actual retention times may vary.

The assay of the invention using the antibodies of the invention also permits detection of additional peaks that presumably correspond to PTH fragments that have intact amino terminal portions.

The HPLC analysis of human serum patient samples, the clinical utility of the bioactive intact N-Terminal anti-PTH₁₋₁₃ antibodies are again shown in demonstrating the specificity of the antibodies to only detect intact PTH 1-84.

## Claims

1. An isolated antibody that recognizes and selectively binds a three-dimensional epitope of parathyroid hormone, wherein the three-dimensional epitope of parathyroid hormone comprises amino acids located between amino acids 1-13 of SEQ ID NO: 1 and includes the first N-terminal amino acid of native PTH along with the intact helix of the amino terminus.

2. The isolated antibody of claim 1, wherein the three-dimensional epitope of parathyroid hormone further includes the second N-terminal amino acid of native PTH.

3. The isolated antibody of claim 1 or 2, wherein the antibody recognizes a peptide comprising or consisting of an amino acid sequence from Ser in the 1 position to Lys in the 13 position of SEQ ID NO: 1.

4. The isolated antibody of any one of claims 1 to 3,
wherein the antibody is effective to reduce adenylate cyclase activity by binding to the bioactive portion of the parathyroid hormone.

5. The isolated antibody of any one of claims 1 to 4,
wherein the three-dimensional epitope comprises a first portion that includes amino acid 1 of SEQ ID NO:1 and a second portion that includes amino acids located between amino acids 10 to 13 of SEQ ID NO:1.

6. The isolated antibody of any one of claims 1 to 5,
wherein the antibody is a polyclonal or monoclonal antibody or an antibody fragment.

7. The isolated antibody of any one of claims 1 to 6 coupled to a detectable marker.

8. A kit comprising an antibody that recognizes and selectively binds a three-dimensional epitope of parathyroid hormone, wherein the three-dimensional epitope of parathyroid hormone comprises amino acids located between amino acids 1-13 of SEQ ID NO: 1 and includes the first N-terminal amino acid of native PTH along with the intact helix of the amino terminus.

9. The kit of claim 8, wherein the three-dimensional epitope comprises a first portion that includes amino acid 1 of SEQ ID NO:1 and a second portion that includes amino acids located between amino acids 10 to 13 of SEQ ID NO:1.

10. The kit of claim 8 or 9, further comprising a detectable label coupled to the antibody.

11. The kit of any one of claims 8 to 10, further comprising tools for obtaining a biological sample containing parathyroid hormone from a patient.

12. The kit of claim 10, wherein the detectable label is selected from the group consisting of chemiluminescent markers, fluorescent markers, radioactive markers, and enzymatic markers.

13. The kit of claim 12, wherein the detectable label is an acridinium ester.

14. A method for detecting bioactive parathyroid hormone in a sample, comprising
a) exposing the sample to an antibody that recognizes and selectively binds a three-dimensional epitope of parathyroid hormone, wherein the three-dimensional epitope of parathyroid hormone comprises amino acids located between amino acids 1-13 of SEQ ID NO: 1 and includes the first N-terminal amino acid of native PTH along with the intact helix of the amino terminus;
b) detecting the antibody-hormone complex, thereby detecting the bioactive parathyroid hormone in the sample.

15. The method of claim 14, wherein the three-dimensional epitope comprises a first portion that includes amino acid 1 of SEQ ID NO:1 and a second portion that includes amino acids located between amino acids 10 to 13 of SEQ ID NO:1.

16. The method of claim 14 or 15, wherein the antibody is coupled to a chemiluminescent marker.

17. The method of claim 16, wherein the chemiluminescent marker is an acridinium ester.

18. The method of any one of claims 14 to 17, wherein the sample is from a patient with hyperparathyroidism.

19. An immunoassay comprising an antibody that recognizes and specifically binds a three-dimensional amino terminus of human parathyroid hormone,
wherein the three-dimensional amino terminus of human parathyroid hormone comprises amino acids located between amino acids 1-13 of SEQ ID NO: 1 and includes the first N-terminal amino acid of native PTH along with the intact helix of the amino terminus.

20. An immunoassay according to claim 19, wherein the three-dimensional amino terminus of human parathyroid hormone comprises a first portion that includes amino acid 1 of SEQ ID NO:1 and a second portion that includes amino acids located between amino acids 10 to 13 of SEQ ID NO:1.

## Patentansprüche

1. Ein isolierter Antikörper der ein drei-dimensionales Epitop von Parathyroidhormon erkennt und selektiv bindet, wobei das drei-dimensionale Epitop von Parathyroidhormon Aminosäuren umfasst, die sich zwischen Aminosäuren 1-13 von SEQ ID NO: 1 befinden und die erste N-terminale Aminosäure von nativem PTH zusammen mit der intakten Helix des Amino-Terminus einschließt.

2. Der isolierte Antikörper nach Anspruch 1, wobei das drei-dimensionale Epitop von Parathyroidhormon ferner die zweite N-terminale Aminosäure von nativem PTH einschließt.

3. Der isolierte Antikörper nach Anspruch 1 oder 2, wobei der Antikörper ein Peptid erkennt, das eine Aminosäuresequenz von Ser in der Position 1 bis Lys in der Position 13 von SEQ ID NO:1 umfasst oder daraus besteht.

4. Der isolierte Antikörper nach einem der Ansprüche 1 bis 3, wobei der Antikörper wirksam ist, um Adenylatcyclase Aktivität durch das Binden an den bioaktiven Teil des Parathyroidhormons zu verringern.

5. Der isolierte Antikörper nach einem der Ansprüche 1 bis 4, wobei das dreidimensionale Epitop einen ersten Teil, der die Aminosäure 1 von SEQ ID NO:1 einschließt, und einen zweiten Teil, der Aminosäuren einschließt, die sich zwischen den Aminosäuren 10 bis 13 von SEQ ID NO:1 befinden, umfasst.

6. Der isolierte Antikörper nach einem der Ansprüche 1 bis 5, wobei der Antikörper ein polyklonaler oder monoklonaler Antikörper oder ein Antikörperfragment ist.

7. Der isolierte Antikörper nach einem der Ansprüche 1 bis 6 gekoppelt an einen nachweisbaren Marker.

8. Ein Kit umfassend einen Antikörper, der ein drei-dimensionales Epitop von Parathyroidhormon erkennt und selektiv bindet, wobei das drei-dimensionale Epitop von Parathyroidhormon Aminosäuren umfasst, die sich zwischen den Aminosäuren 1-13 von SEQ ID NO: 1 befinden und die erste N-terminale Aminosäure von nativem PTH zusammen mit der intakten Helix des Amino-Terminus einschließt.

9. Der Kit nach Anspruch 8, wobei das drei-dimensionale Epitop einen ersten Teil, der Aminosäure 1 von SEQ ID NO:1 einschließt, und einen zweiten Teil, der Aminosäuren einschließt, die sich zwischen Aminosäuren 10-13 von SEQ ID NO:1 befinden, umfasst.

10. Der Kit nach Anspruch 8 oder 9, ferner umfassend einen nachweisbaren Marker, der an den Antikörper gekoppelt ist.

11. Der Kit nach einem der Ansprüche 8 bis 10, ferner umfassend Mittel um einem Patienten eine biologische Probe, die Parathyroidhormon enthält, zu entnehmen.

12. Der Kit nach Anspruch 10, wobei der nachweisbare Marker ausgewählt wird aus der Gruppe bestehend aus chemolumineszenten Markern, fluoreszenten Markern, radioaktiven Markern und enzymatischen Markern.

13. Der Kit nach Anspruch 12, wobei der nachweisbare Marker ein Acridiniumester ist.

14. Ein Verfahren zum Nachweis von bioaktiven Parathyroidhormon in einer Probe, umfassend
a) Aussetzen der Probe gegenüber einem Antikörper, der ein drei-dimensionales Epitop von Parathyroidhormon erkennt und selektiv bindet, wobei das drei-dimensionale Epitop von Parathyroidhormon Aminosäuren umfasst, die sich zwischen Aminosäuren 1-13 von SEQ ID NO:1 befinden und die erste N-terminale Aminosäure von nativem PTH zusammen mit der intakten Helix des Amino-Terminus einschließt;
b) Nachweisen des Antikörper-Hormonkomplexes und **dadurch** Nachweisen des bioaktiven Parathyroidhormons in der Probe.

15. Verfahren nach Anspruch 14, wobei das drei-dimensionale Epitop einen ersten Teil, der Aminosäure 1 von SEQ ID NO:1 einschließt, und einen zweiten Teil, der Aminosäuren einschließt, die sich zwischen den Aminosäuren 10-13 von SEQ ID NO:1 befinden, umfasst.

16. Das Verfahren nach Anspruch 14 oder 15, wobei der Antikörper an einen chemolumineszenten Marker gekoppelt ist.

17. Das Verfahren nach Anspruch 16, wobei der chemolumineszente Marker ein Acridiniumester ist.

18. Das Verfahren nach einem der Ansprüche 14 bis 17, wobei die Probe aus einem Patienten mit Hyperparathyroidismus stammt.

19. Ein Immunassay umfassend einen Antikörper, der einen drei-dimensionalen Amino Terminus von humanem Parathyroidhormon erkennt und spezifisch bindet, wobei der drei-dimensionale Amino-Terminus von humanem Parathyroidhormon Aminosäuren umfasst, die sich zwischen Aminosäuren 1-13 von SEQ ID NO:1 befinden, und die ersten N-terminale Aminosäure von nativem PTH zusammen mit der intakten Helix des Amino-Terminus einschließt.

20. Ein Immunassay gemäß Anspruch 19, wobei der drei-dimensionale Amino-Terminus von humanem Parathyroidhormon einen ersten Teil, der Aminosäure 1 von SEQ ID NO:1 einschließt, und einen zweiten Teil, der Aminosäuren einschließt, die sich zwischen Aminsäuren 10-13 von SEQ ID NO:1 befinden, umfasst.

## Revendications

1. Anticorps isolé qui reconnaît et se lie de manière sélective à un épitope tridimensionnel d'une hormone parathyroïdienne, où l'épitope tridimensionnel de l'hormone parathyroïdienne comprend des acides aminés situés entre les acides aminés 1 à 13 de la SEQ ID NO: 1 et comprend le premier acide aminé N-terminal de la PTH native ainsi que l'hélice intacte de la terminaison amine.

2. Anticorps isolé selon la revendication 1, où l'épitope tridimensionnel de l'hormone parathyroïdienne comprend en outre le deuxième acide aminé N-terminal de la PTH native.

3. Anticorps isolé selon la revendication 1 ou 2, où l'anticorps reconnaît un peptide comprenant ou consistant en une séquence d'acides aminés à partir de la Ser à la position 1 jusqu'à la Lys à la position 13 de la SEQ ID NO: 1.

4. Anticorps isolé selon l'une quelconque des revendications 1 à 3, où l'anticorps est efficace pour réduire l'activité adénylate cyclase en se liant à la partie bioactive de l'hormone parathyroïdienne.

5. Anticorps isolé selon l'une quelconque des revendications 1 à 4, où l'épitope tridimensionnel comprend une première partie qui inclut l'acide aminé 1 de la SEQ ID NO: 1 et une deuxième partie qui inclut les acides aminés situés entre les acides aminés 10 à 13 de la SEQ ID NO: 1.

6. Anticorps isolé selon l'une quelconque des revendications 1 à 5, où l'anticorps est un anticorps polyclonal ou monoclonal ou un fragment d'anticorps.

7. Anticorps isolé selon l'une quelconque des revendications 1 à 6 couplé à un marqueur détectable.

8. Nécessaire comprenant un anticorps qui reconnaît et se lie de manière sélective à un épitope tridimensionnel d'une hormone parathyroïdienne, où l'épitope tridimensionnel de l'hormone parathyroïdienne comprend des acides aminés situés entre les acides aminés 1 à 13 de la SEQ ID NO: 1 et inclut le premier acide aminé N-terminal de la PTH native ainsi que l'hélice intacte de la terminaison amine.

9. Nécessaire selon la revendication 8, où l'épitope tridimensionnel comprend une première partie qui inclut l'acide aminé 1 de la SEQ ID NO: 1 et une deuxième partie qui inclut les acides aminés situés entre les acides aminés 10 à 13 de la SEQ ID NO: 1.

10. Nécessaire selon les revendications 8 ou 9, comprenant en outre un traceur détectable couplé à l'anticorps.

11. Nécessaire selon l'une quelconque des revendications 8 à 10, comprenant en outre des outils pour obtenir un échantillon biologique d'un patient contenant une hormone parathyroïdienne.

12. Nécessaire selon la revendication 10, où le traceur détectable est choisi dans le groupe consistant en marqueurs chimioluminescents, marqueurs fluorescents, marqueurs radioactifs, et marqueurs enzymatiques.

13. Nécessaire selon la revendication 12, où le traceur détectable est un ester d'acridinium.

14. Procédé servant à détecter une hormone parathyroïdienne bioactive dans un échantillon, comprenant :
a) l'exposition de l'échantillon à un anticorps qui reconnaît et se lie de manière sélective à un épitope tridimensionnel d'une hormone parathyroïdienne, où l'épitope tridimensionnel de l'hormone parathyroïdienne comprend des acides aminés situés entre les acides aminés 1 à 13 de la SEQ ID NO: 1 et inclut le premier acide aminé N-terminal de la PTH native ainsi que l'hélice intacte de la terminaison amine ;
b) la détection du complexe anticorps-hormone, en détectant ainsi l'hormone parathyroïdienne bioactive dans l'échantillon.

15. Procédé selon la revendication 14, où l'épitope tridimensionnel comprend une première partie qui inclut l'acide aminé 1 de la SEQ ID NO: 1 et une deuxième partie qui inclut les acides aminés situés entre les acides aminés 10 à 13 de la SEQ ID NO: 1.

16. Procédé selon la revendication 14 ou 15, où l'anticorps est couplé à un marqueur chimioluminescent.

17. Procédé selon la revendication 16, où le marqueur chimioluminescent est un ester d'acridinium.

18. Procédé selon l'une quelconque des revendications 14 à 17, où l'échantillon provient d'un patient atteint d'hyperparathyroïdie.

19. Immunoessai comprenant un anticorps qui reconnaît et se lie de manière spécifique à une extrémité amino terminale tridimensionnelle d'une hormone parathyroïdienne humaine,
où la terminaison amine tridimensionnelle de l'hormone parathyroïdienne humaine comprend les acides aminés situés entre les acides aminés 1 à 13 de la SEQ ID NO: 1 et inclut le premier acide aminé N-terminal de la PTH native ainsi que l'hélice intacte de la terminaison amine.

20. Immunoessai selon la revendication 19, où la terminaison amine tridimensionnelle de l'hormone parathyroïdienne humaine comprend une première partie qui inclut l'acide aminé 1 de la SEQ ID NO: 1 et une deuxième partie qui inclut les acides aminés situés entre les acides aminés 10 à 13 de la SEQ ID NO: 1.


### SEQUENCE LISTING

<110> Hutchison, James Scott
<120> PARATHYROID HORMONE ANTIBODIES AND RELATED METHODS
<130> A1713
<160> 8
<170> PatentIn version 3.1
<210> 1
   <211> 84
   <212> PRT
   <213> Homo sapiens
<400> 1
   Ser Val Ser Glu Ile Gln Leu Met His Asn Leu Gly Lys His Leu Asn
   1               5                   10                  15
   Ser Met Glu Arg Val Glu Trp Leu Arg Lys Lys Leu Gln Asp Val His
               20                  25                  30
   Asn Phe Val Ala Leu Gly Ala Pro Leu Ala Pro Arg Asp Ala Gly Ser
           35                  40                  45
   Gln Arg Pro Arg Lys Lys Glu Asp Asn Val Leu Val Glu Ser His Glu
       50                  55                  60
   Lys Ser Leu Gly Glu Ala Asp Lys Ala Asp Val Asn Val Leu Thr Lys
   65                  70                  75                  80
   Ala Lys Ser Gln
<210> 2
   <211> 13
   <212> PRT
   <213> Homo sapiens
<400> 2
   Ala Val Ser Glu Ile Gln Phe Met His Asn Leu Gly Lys
   1 5 10
<210> 3
   <211> 13
   <212> PRT
   <213> Homo sapiens
<400> 3
   Ala Val Ser Glu Ile Gln Leu Met His Asn Leu Gly Lys
   1 5 10
<210> 4
   <211> 13
   <212> PRT
   <213> Homo sapiens
<400> 4
   Ser Val Ser Glu Ile Gln Leu Met His Asn Leu Gly Lys
   1 5 10
<210> 5
   <211> 13
   <212> PRT
   <213> Homo sapiens
<400> 5
   Ser Val Ser Glu Ile Gln Leu Cys His Asn Leu Gly Lys
   1 5 10
<210> 6
   <211> 13
   <212> PRT
   <213> Homo sapiens
<400> 6
   Ala Val Ser Glu Ile Gln Phe Cys His Asn Leu Gly Lys
   1 5 10
<210> 7
   <211> 13
   <212> PRT
   <213> Homo sapiens
<400> 7
   Ala Val Ser Glu Ile Gln Leu Cys His Asn Leu Gly Lys
   1 5 10
<210> 8
   <211> 13
   <212> PRT
   <213> Homo sapiens
<400> 8
   Ser Val Ser Glu Ile Gln Leu Cys His Asn Leu Gly Lys
   1 5 10